(19) Europäisches Patentamt
European Patent Office
Office européen des brevets

(11) EP 2 762 141 A1

(12) **EUROPEAN PATENT APPLICATION**
published in accordance with Art. 153(4) EPC

(43) Date of publication:
**06.08.2014 Bulletin 2014/32**

(21) Application number: **12836575.6**

(22) Date of filing: **28.09.2012**

(51) Int Cl.:
***A61K 31/496*** *(2006.01)* ***A61K 31/445*** *(2006.01)*
***A61K 31/4545*** *(2006.01)* ***A61K 31/495*** *(2006.01)*
***A61P 11/02*** *(2006.01)* ***A61P 37/08*** *(2006.01)*
***A61P 43/00*** *(2006.01)*

(86) International application number:
**PCT/JP2012/075030**

(87) International publication number:
**WO 2013/047725 (04.04.2013 Gazette 2013/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.09.2011 JP 2011214083**
**27.10.2011 JP 2011236213**

(71) Applicant: **Shionogi & Co., Ltd.**
**Osaka-shi, Osaka 541-0045 (JP)**

(72) Inventors:
- **SAWADA, Takuko**
  **Osaka-shi**
  **Osaka 553-0002 (JP)**
- **ARIMURA, Akinori**
  **Osaka-shi**
  **Osaka 553-0002 (JP)**
- **KUWAJIMA, Goro**
  **Osaka-shi**
  **Osaka 553-0002 (JP)**

(74) Representative: **Vossius & Partner**
**Siebertstrasse 4**
**81675 München (DE)**

# (54) DRUG FOR THE TREATMENT OF ALLERGIC RHINITIS COMPRISING PGD2 ANTAGONIST AND HISTAMINE ANTAGONIST

(57) A medicament for treating allergic rhinitis, characterized in that (A) a compound represented by the formula (I) or a pharmaceutically acceptable salt thereof, is combined with (B) at least one compound selected from the group consisting of cetirizine, fexofenadine, and loratadine, or a pharmaceutically acceptable salt thereof. Since the effects of the medicament for treating allergic rhinitis of the present invention surpass the sum of the effects of single dose of each of the agents against the allergic rhinitis, the medicament is useful as a medicament for treating allergic rhinitis having potent effects.



Printed by Jouve, 75001 PARIS (FR)

## Description

### TECHNICAL FIELD

The present invention relates to a medicament for treating allergic rhinitis. More specifically, the present invention relates to a medicament for treating allergic rhinitis characterized in that a particular PGD2 receptor antagonist is combined with a particular histamine H1 receptor antagonist are combined.

### BACKGROUND ART

Prostaglandin D2 (PGD2), which is a product of a cyclooxygenase pathway for metabolism of arachidonic acid has a potent bronchoconstricting action, causing an increase in vascular permeability and migration of inflammatory cells such as eosinophils. Therefore, PGD2 receptor antagonists have been known to be useful in the treatment of allergic diseases, for example, asthma, allergic rhinitis, allergic dermatitis, allergic conjunctivitis, and the like.

For example, the Applicant of the present invention has reported a sulfonamide derivative having antagonistic activity to DP receptor, one of the PGD2 receptors, as a therapeutic agent for allergic diseases (see, Patent Publication 1).

In addition, it has been reported in Patent Publication 2 that a PGD2 receptor antagonist is used together with a leukotriene (LT) receptor antagonist, which is similarly a product of a 5-lipoxygenase pathway for metabolism of arachidonic acid, so that ingredients showing different action mechanisms against asthma are used together, thereby showing additive effects against anti-asthma.

It has been reported in Patent Publication 3 that a combined use of a PGD2 receptor antagonist with a histamine H1 receptor antagonist and/or an LT receptor antagonist is useful for allergic diseases, and mepyramine has been used as an antihistamine.

In the treatment of allergic rhinitis, an oral or intranasal histamine H1 receptor antagonist, an intranasal steroid, or an LT receptor antagonist or the like has been used. In addition, studies on the combined use of the medicaments for treating allergic rhinitis having these different mechanisms have also been made.

For example, Journal of Asthma, 46, 878-883, 2009 (Non-Patent Publication 1) describes clinical test results (Ph 2, protocol 077) on seasonal allergic rhinitis for a patient group administered with montelukast as an LT receptor antagonist, a patient group administered with loratadine as a histamine H1 receptor antagonist, and a patient group administered with beclomethasone as an intranasal steroid (see Table 3 in the publication). Here, the nasal symptoms, i.e. sneezing, nasal discharge, nasal congestion, and nasal itching, after two weeks since the start of the oral administration are scored to be evaluated. A change in scores per symptom from the placebo group is as follows: The change in a patient group administered with montelukast is 0.14, i.e. a difference between 0.36, a change in the patient group administered with montelukast since the start of the administration, and 0.22, a change in the placebo group since the start of the administration; and the change in a patient group administered with loratadine 0.31, i.e. a difference between 0.53, a change in the patient group administered with loratadine since the start of the administration, and 0.22, a change in the placebo group since the start of the administration, so that the changes were significantly larger as compared to the placebo group. On the other hand, an extent of the effect exhibited by the combined use was not sufficient. In the group with combination administration of montelukast and loratadine, a change per symptom from the placebo group is 0.32, i.e. a difference between 0.54 and 0.22, and not meeting the level of 0.45, a sum of changes when each of the two agents is administered alone, so that it can be seen that additive effects are not exhibited.

Further, Non-Patent Publication 1 discloses the clinical test results (Ph 2, protocol 102) on seasonal allergic rhinitis in the same manner (see, Table 6 of the publication). Also in this test, if a change per symptom of the nasal symptom scores after two weeks since the start of the oral administration, based on the placebo group, is calculated in the same manner, the change is as follows: The change in a patient group administered with montelukast is 0.08, i.e. a difference between 0.39 and 0.31; the change in a patient group administered with loratadine is 0.09, i.e. a difference between 0.40 and 0.31; the change in a patient group with combination administration is 0.19, i.e. a difference between 0.50 and 0.31, so that a sum of changes in the patient group administered with montelukast and the patient group administered with loratadine is nearly of the same level as the patient group with combination administration, so that it could be understood that additive effects are exhibited. However, in this test, a change per symptom of the nasal symptom scores after two weeks since the start of the oral administration, based on the placebo group, for a patient group administered with loratadine is 0.09, which is markedly lower than an assumed change in loratadine in an ordinary test, so that it is thought that the test lacks reliability.

In addition, Journal of Allergy and Clinical Immunology, 127(5), 917-922, 2000 (Non-Patent Publication 2) describes clinical test results (Ph 2) on seasonal allergic rhinitis for a patient group administered with 10 mg or 20 mg of montelukast as an LT receptor antagonist, a patient group administered with 10 mg of loratadine as a histamine H1 receptor antagonist, and a patient group with combination administration of 10 mg each thereof (see, Table III of the publication). According to the description, a change per symptom of the nasal symptom scores after two weeks since

the start of the oral administration, based on the placebo group, is as follows: The change in a patient group administered with 10 mg of montelukast is 0.11, i.e. a difference between 0.36 and 0.25, the change in a patient group administered with 10 mg of loratadine is 0.09, i.e. a difference between 0.34 and 0.25, and the change in a patient group with combination administration is 0.36, i.e. a difference between 0.61 and 0.25. The sum of changes in the patient group administered with 10 mg of montelukast and the patient group administered with 10 mg of loratadine is 0.20, so that a change for the patient group with combination administration of 0.36 could be understood to exhibit effects surpassing the additive effects. However, in this test, a change per symptom of the nasal symptom scores after two weeks since the start of the oral administration, based on the placebo group, for a patient group administered with loratadine is 0.09, which is markedly lower than an assumed change in loratadine in an ordinary test, so that it is thought that the test lacks reliability.

As described above, it is obvious that an effect of combined use of montelukast as an LT receptor antagonist and loratadine as a histamine H1 receptor antagonist does not show an effect that is an additive effect or higher of a case where each is administered alone.

As described above, anti-allergic agents having different mechanisms are combination-administered to human, an effect of additive effects for each agent, in other words, synergistic effects, cannot be usually observed.

PRIOR ART PUBLICATIONS

PATENT PUBLICATIONS

Patent Publication 1: WO 2007/037187 Pamphlet
Patent Publication 2: WO 2006/118169 Pamphlet
Patent Publication 3: WO 2001/078697 Pamphlet

NON-PATENT PUBLICATIONS

Non-Patent Publication 1: Journal of Asthma, 46, 878-883, 2009
Non-Patent Publication 2: Journal of Allergy and Clinical Immunology, 127(5), 917-922, 2000

SUMMARY OF THE INVENTION

PROBLEMS TO BE SOLVED BY THE INVENTION

An object of the present invention is to provide a medicament for treating allergic rhinitis showing even more potent pharmacological actions by a combined use of a PGD2 receptor antagonist and a histamine H1 receptor antagonist.

MEANS TO SOLVE THE PROBLEMS

As a result of intensive studies in order to solve the above problems, the present inventors have newly found that symptoms of rhinitis (nasal congestion, sneezing, and nasal discharge) are more potently suppressed by a combined use of a specified compound (compound II-74) among the PGD2 receptor antagonists described in Patent Publication 1, and a specified histamine H1 receptor antagonist, as compared to cases where the agents are administered alone, that those effects exhibit substantially maximal pharmacological efficacy within two days since the start of the administration, and further that those effects exhibit in orally administered agent are the same level as those of intranasal steroid agent having the most potent effects as an anti-allergic agent. The present invention has been perfected thereby.

Specifically, the present invention relates to:

(1) a medicament for treating allergic rhinitis, characterized in that a compound represented by the formula (I):

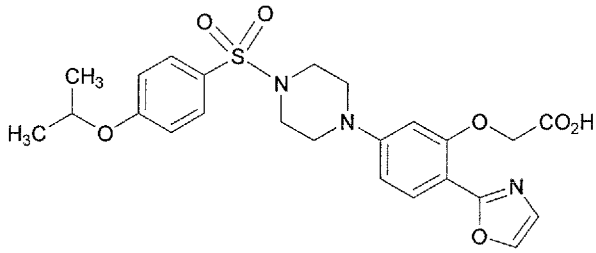

(I)

or a pharmaceutically acceptable salt thereof, is combined with at least one compound selected from the group consisting of cetirizine, fexofenadine, and loratadine, or a pharmaceutically acceptable salt thereof.
More specifically, the present invention relates to the following (2) to (38):
(2) the medicament according to the above (1), wherein the allergic rhinitis is perennial allergic rhinitis or seasonal allergic rhinitis;
(3) the medicament according to the above (1) or (2), wherein the medicament reaches substantially maximum pharmacological efficacy for nasal congestion, sneezing, or nasal discharge, within two days since the start of administration;
(4) the medicament according to any one of the above (1) to (3), wherein the medicament is an orally administered agent;
(5) the medicament according to any one of the above (1) to (4), characterized in that 10 to 200 mg of the compound represented by the formula (I) or a pharmaceutically acceptable salt thereof is combined with 2.5 to 10 mg of cetirizine or a pharmaceutically acceptable salt thereof;
(6) the medicament according to any one of the above (1) to (4), characterized in that 50 to 100 mg of the compound represented by the formula (I) or a pharmaceutically acceptable salt thereof is combined with 5 to 10 mg of cetirizine or a pharmaceutically acceptable salt thereof;
(7) the medicament according to any one of the above (1) to (4), characterized in that 10 to 100 mg of the compound represented by the formula (I) or a pharmaceutically acceptable salt thereof is combined with 5 to 10 mg of cetirizine or a pharmaceutically acceptable salt thereof;
(8) the medicament according to any one of the above (1) to (4), characterized in that 10 to 100 mg of the compound represented by the formula (I) is combined with 5 to 10 mg of cetirizine hydrochloride;
(9) the medicament according to any one of the above (1) to (4), characterized in that 10 to 200 mg of the compound represented by the formula (I) or a pharmaceutically acceptable salt thereof is combined with 30 to 120 mg of fexofenadine or a pharmaceutically acceptable salt thereof;
(10) the medicament according to any one of the above (1) to (4), characterized in that 50 to 100 mg of the compound represented by the formula (I) or a pharmaceutically acceptable salt thereof is combined with 60 to 120 mg of fexofenadine or a pharmaceutically acceptable salt thereof;
(11) the medicament according to any one of the above (1) to (4), characterized in that 10 to 200 mg of the compound represented by the formula (I) or a pharmaceutically acceptable salt thereof is combined with 2.5 to 10 mg of loratadine or a pharmaceutically acceptable salt thereof;
(12) the medicament according to any one of the above (1) to (4), characterized in that 50 to 100 mg of the compound represented by the formula (I) or a pharmaceutically acceptable salt thereof is combined with 5 to 10 mg of loratadine or a pharmaceutically acceptable salt thereof;
(13) a combination product for treating allergic rhinitis, containing a compound represented by the formula (I):

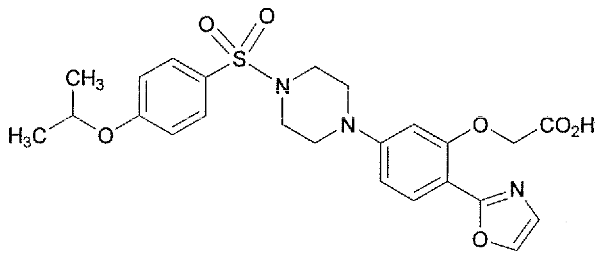

(I)

or a pharmaceutically acceptable salt thereof, and at least one compound selected from the group consisting of cetirizine, fexofenadine, and loratadine, or a pharmaceutically acceptable salt thereof;
(14) the combination product for treating allergic rhinitis according to the above (13), wherein the allergic rhinitis is

perennial allergic rhinitis or seasonal allergic rhinitis;
(15) the combination product for treating allergic rhinitis according to the above (13) or (14), wherein the combination product reaches substantially maximum pharmacological efficacy for nasal congestion, sneezing, or nasal discharge, within two days since the start of administration;
(16) the combination product for treating allergic rhinitis according to any one of the above (13) to (15), wherein the combination product is an orally administered agent;
(17) the combination product for treating allergic rhinitis according to any one of the above (13) to (16), containing 10 to 200 mg of the compound represented by the formula (I) or a pharmaceutically acceptable salt thereof, and 2.5 to 10 mg of cetirizine or a pharmaceutically acceptable salt thereof;
(18) the combination product for treating allergic rhinitis according to any one of the above (13) to (16), containing 50 to 100 mg of the compound represented by the formula (I) or a pharmaceutically acceptable salt thereof, and 5 to 10 mg of cetirizine or a pharmaceutically acceptable salt thereof;
(19) the combination product for treating allergic rhinitis according to any one of the above (13) to (16), containing 10 to 100 mg of the compound represented by the formula (I) or a pharmaceutically acceptable salt thereof, and 5 to 10 mg of cetirizine or a pharmaceutically acceptable salt thereof;
(20) the combination product for treating allergic rhinitis according to any one of the above (13) to (16), containing 10 to 100 mg of the compound represented by the formula (I), and 5 to 10 mg of cetirizine hydrochloride;
(21) the combination product for treating allergic rhinitis according to any one of the above (13) to (16), containing 10 to 200 mg of the compound represented by the formula (I) or a pharmaceutically acceptable salt thereof, and 30 to 120 mg of fexofenadine or a pharmaceutically acceptable salt thereof;
(22) the combination product for treating allergic rhinitis according to any one of the above (13) to (16), containing 50 to 100 mg of the compound represented by the formula (I) or a pharmaceutically acceptable salt thereof, and 60 to 120 mg of fexofenadine or a pharmaceutically acceptable salt thereof;
(23) the combination product according to any one of the above (13) to (16), containing 10 to 200 mg of the compound represented by the formula (I) or a pharmaceutically acceptable salt thereof, and 2.5 to 10 mg of loratadine or a pharmaceutically acceptable salt thereof;
(24) the combination product for treating allergic rhinitis according to any one of the above (13) to (16), containing 50 to 100 mg of the compound represented by the formula (I) or a pharmaceutically acceptable salt thereof, and 5 to 10 mg of loratadine or a pharmaceutically acceptable salt thereof;
(25) a method for treating for allergic rhinitis, including the steps of administering in combination a compound represented by the formula (I):

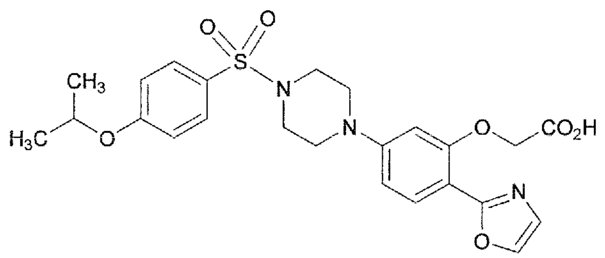

(I)

,

or a pharmaceutically acceptable salt thereof, and at least one compound selected from the group consisting of cetirizine, fexofenadine, and loratadine, or a pharmaceutically acceptable salt thereof in a therapeutically effective amount thereof to an individual in need of treatment for allergic rhinitis;
(26) the method for treatment according to the above (25), wherein the allergic rhinitis is perennial allergic rhinitis or seasonal allergic rhinitis;
(27) the method for treatment according to the above (25) or (26), wherein a substantially maximum pharmacological efficacy for nasal congestion, sneezing, or nasal discharge is attained, within two days since the start of administration;
(28) the method for treatment according to any one of the above (25) to (27), wherein the administration is carried out orally;
(29) a pharmaceutical composition for use in the treatment of allergic rhinitis, containing a compound represented by the formula (I):

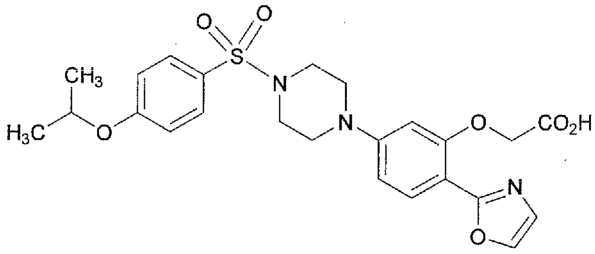

(I)

,

or a pharmaceutically acceptable salt thereof, in combination with at least one compound selected from the group consisting of cetirizine, fexofenadine, and loratadine, or a pharmaceutically acceptable salt thereof;
(30) a combination for use in the treatment of allergic rhinitis, containing a compound represented by the formula (I):

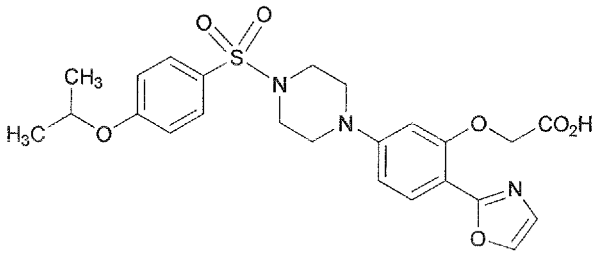

(I)

,

or a pharmaceutically acceptable salt thereof, and
at least one compound selected from the group consisting of cetirizine, fexofenadine, and loratadine, or a pharmaceutically acceptable salt thereof;
(31) the pharmaceutical composition according to the above (29), wherein the allergic rhinitis is perennial allergic rhinitis or seasonal allergic rhinitis;
(32) the combination according to the above (30), wherein the allergic rhinitis is perennial allergic rhinitis or seasonal allergic rhinitis;
(33) a compound represented by the formula (I):

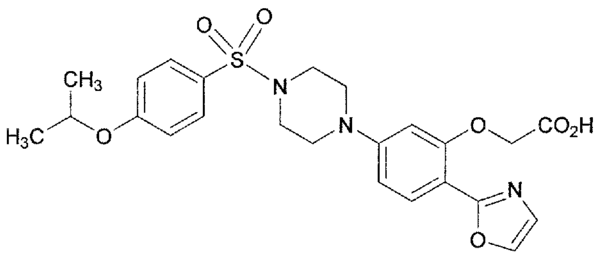

(I)

,

or a pharmaceutically acceptable salt thereof, for use in the treatment of allergic rhinitis, wherein the compound or a pharmaceutically acceptable salt thereof is used together with at least one compound selected from the group consisting of cetirizine, fexofenadine, and loratadine, or a pharmaceutically acceptable salt thereof.
(34) the compound or a pharmaceutically acceptable salt thereof according to the above (33), wherein the allergic rhinitis is perennial allergic rhinitis or seasonal allergic rhinitis;
(35) use of a composition in the manufacture of a medicament for treating allergic rhinitis, wherein the composition contains a compound represented by the formula (I):

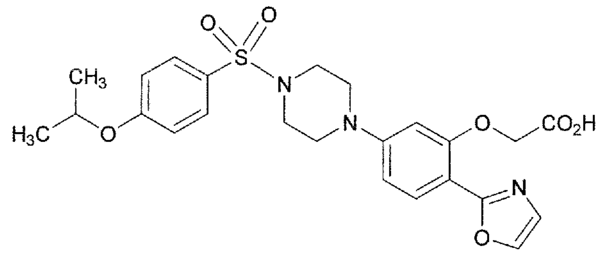

(I)

,

or a pharmaceutically acceptable salt thereof, in combination with at least one compound selected from the group consisting of cetirizine, fexofenadine, and loratadine, or a pharmaceutically acceptable salt thereof;

(36) the use according to the above (35), wherein the allergic rhinitis is perennial allergic rhinitis or seasonal allergic rhinitis;

(37) a pharmaceutical formulation for use in the treatment of allergic rhinitis containing, in a single dosage form or separate dosage forms, a compound represented by the formula (I):

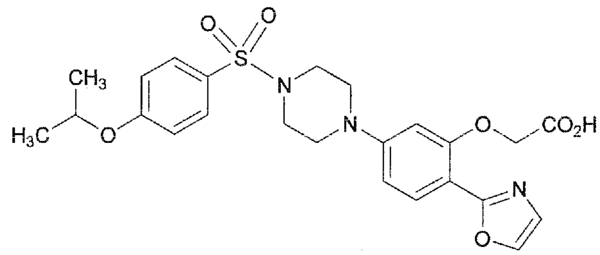

(I)

,

or a pharmaceutically acceptable salt thereof, in combination with at least one compound selected from the group consisting of cetirizine, fexofenadine, and loratadine, or a pharmaceutically acceptable salt thereof; and

(38) a kit for treating allergic rhinitis, containing a compound represented by the formula (I):

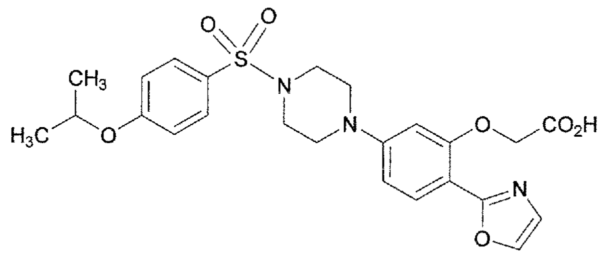

(I)

,

or a pharmaceutically acceptable salt thereof, and

at least one compound selected from the group consisting of cetirizine, fexofenadine, and loratadine, or a pharmaceutically acceptable salt thereof.

More specifically, the present invention relates to the following (I) to (XX).

(I) a therapeutic agent for allergic rhinitis, characterized in that a compound represented by the formula (I):

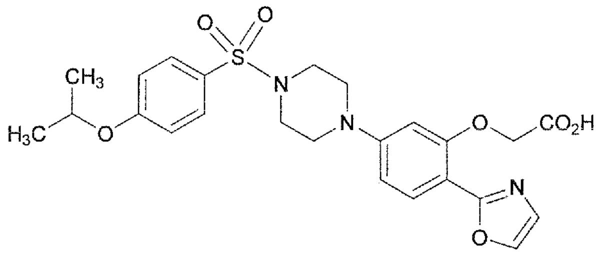

(I)

,

or a pharmaceutically acceptable salt thereof is combined with at least one compound selected from the group consisting of cetirizine, fexofenadine, and loratadine, or a pharmaceutically acceptable salt thereof;

(II) the therapeutic agent according to the above (I), wherein the allergic rhinitis is perennial allergic rhinitis or seasonal allergic rhinitis;

(III) the therapeutic agent according to the above (I) or (II), wherein the therapeutic agent reaches substantially maximum pharmacological efficacy for nasal congestion, sneezing, or nasal discharge, within two days since the start of administration;

(IV) the therapeutic agent according to any one of the above (I) to (III), wherein the therapeutic agent is an orally administered agent;

(V) the therapeutic agent according to any one of the above (I) to (IV), characterized in that 10 to 200 mg of the compound represented by the formula (I) or a pharmaceutically acceptable salt thereof is combined with 2.5 to 10 mg of cetirizine or a pharmaceutically acceptable salt thereof;

(VI) the therapeutic agent according to any one of the above (I) to (IV), characterized in that 50 to 100 mg of the compound represented by the formula (I) or a pharmaceutically acceptable salt thereof is combined with 5 to 10 mg of cetirizine or a pharmaceutically acceptable salt thereof;

(VII) the therapeutic agent according to any one of the above (I) to (IV), characterized in that 10 to 200 mg of the compound represented by the formula (I) or a pharmaceutically acceptable salt thereof is combined with 30 to 120 mg of fexofenadine or a pharmaceutically acceptable salt thereof;

(VIII) the therapeutic agent according to any one of the above (I) to (IV), characterized in that 50 to 100 mg of the compound represented by the formula (I) or a pharmaceutically acceptable salt thereof is combined with 60 to 120 mg of fexofenadine or a pharmaceutically acceptable salt thereof;

(IX) the therapeutic agent according to any one of the above (I) to (IV), characterized in that 10 to 200 mg of the compound represented by the formula (I) or a pharmaceutically acceptable salt thereof is combined with 2.5 to 10 mg of loratadine or a pharmaceutically acceptable salt thereof;

(X) the therapeutic agent according to any one of the above (I) to (IV), characterized in that 50 to 100 mg of the compound represented by the formula (I) or a pharmaceutically acceptable salt thereof is combined with 5 to 10 mg of loratadine or a pharmaceutically acceptable salt thereof;

(XI) a combination product for treating allergic rhinitis, containing a compound represented by the formula (I):

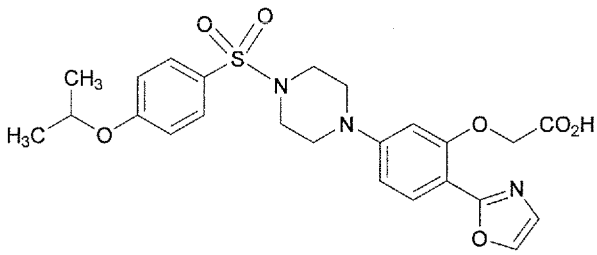

(I)

,

or a pharmaceutically acceptable salt thereof, and

at least one compound selected from the group consisting of cetirizine, fexofenadine, and loratadine, or a pharmaceutically acceptable salt thereof;

(XII) the combination product for treating allergic rhinitis according to the above (XI), wherein the allergic rhinitis is perennial allergic rhinitis or seasonal allergic rhinitis;

(XIII) the combination product for treating allergic rhinitis according to the above (XI) or (XII), wherein the combination product reaches substantially maximum pharmacological efficacy for nasal congestion, sneezing, or nasal discharge, within two days since the start of administration;

(XIV) the combination product for treating allergic rhinitis according to any one of the above (XI) to (XIII), wherein

the combination product is an orally administered agent;
(XV) the combination product for treating allergic rhinitis according to any one of the above (XI) to (XIV), characterized in that the combination product contains 10 to 200 mg of the compound represented by the formula (I) or a pharmaceutically acceptable salt thereof, and 2.5 to 10 mg of cetirizine or a pharmaceutically acceptable salt thereof;
(XVI) the combination product for treating allergic rhinitis according to any one of the above (XI) to (XIV), characterized in that the combination product contains 50 to 100 mg of the compound represented by the formula (I) or a pharmaceutically acceptable salt thereof, and 5 to 10 mg of cetirizine or a pharmaceutically acceptable salt thereof;
(XVII) the combination product for treating allergic rhinitis according to any one of the above (XI) to (XIV), characterized in that the combination product contains 10 to 200 mg of the compound represented by the formula (I) or a pharmaceutically acceptable salt thereof, and 30 to 120 mg of fexofenadine or a pharmaceutically acceptable salt thereof;
(XVIII) the combination product for treating allergic rhinitis according to any one of the above (XI) to (XIV), characterized in that the combination product contains 50 to 100 mg of the compound represented by the formula (I) or a pharmaceutically acceptable salt thereof, and 60 to 120 mg of fexofenadine or a pharmaceutically acceptable salt thereof;
(XIX) the combination product for treating allergic rhinitis according to any one of the above (XI) to (XIV), characterized in that the combination product contains 10 to 200 mg of the compound represented by the formula (I) or a pharmaceutically acceptable salt thereof, and 2.5 to 10 mg of loratadine or a pharmaceutically acceptable salt thereof; and
(XX) the combination product for treating allergic rhinitis according to any one of the above (XI) to (XIV), characterized in that the combination product contains 50 to 100 mg of the compound represented by the formula (I) or a pharmaceutically acceptable salt thereof, and 5 to 10 mg of loratadine or a pharmaceutically acceptable salt thereof.

## EFFECTS OF THE INVENTION

The medicament for treating allergic rhinitis of the present invention exhibits some excellent effects that symptoms of rhinitis (nasal congestion, sneezing, nasal discharge) are suppressed to a level of additive effect or more, as compared to cases where each of a PGD2 receptor antagonist, a compound represented by the formula (I) or a pharmaceutically acceptable salt thereof, or cetirizine, fexofenadine, and loratadine, which are histamine H1 receptor antagonists, or a pharmaceutically acceptable salt thereof, is administered alone, that those effects exhibit substantially maximal pharmacological efficacy within two days since the start of the administration, and further that those effects exhibit in orally administered agent are the same level as those of intranasal steroid agent having the most potent effects as an anti-allergic agent.

## BRIEF DESCRIPTION OF THE DRAWINGS

[Figure 1] A graph showing a change in an average of a total of three nasal symptom scores, i.e. nasal congestion, sneezing, and nasal discharge, on a weekly basis.
[Figure 2] A graph showing a change in a total of three nasal symptom scores, i.e. nasal congestion, sneezing, and nasal discharge, on a daily basis.
[Figure 3] A graph showing a change in an average of sneezing scores, on a weekly basis.
[Figure 4] A graph showing a change in sneezing scores, on a daily basis.
[Figure 5] A graph showing a change in an average of nasal discharge scores, on a weekly basis.
[Figure 6] A graph showing a change in nasal discharge scores, on a daily basis.
[Figure 7] A graph showing a change in an average of nasal congestion scores, on a weekly basis.
[Figure 8] A graph showing a change in nasal congestion scores, on a daily basis.
[Figure 9] A graph showing a change in an average of nasal itching scores, on a weekly basis.
[Figure 10] A graph showing a change in nasal itching, on a daily basis.
[Figure 11] A graph showing a changed proportion of nasal airway resistance according to Evaluation Test 1 for pharmacological effects of guinea pig rhinitis model.
[Figure 12] A graph showing a changed proportion of nasal airway resistance according to Evaluation Test 2 for pharmacological effects of guinea pig rhinitis model.
[Figure 13] A graph showing a changed proportion of nasal airway resistance according to Evaluation Test 3 for pharmacological effects of guinea pig rhinitis model induced with PGD2 and histamine.

## MODES FOR CARRYING OUT THE INVENTION

The medicament for treating allergic rhinitis of the present invention is characterized by a combination use

(including a kit), as active ingredients, of

(A) a compound represented by the formula (I):

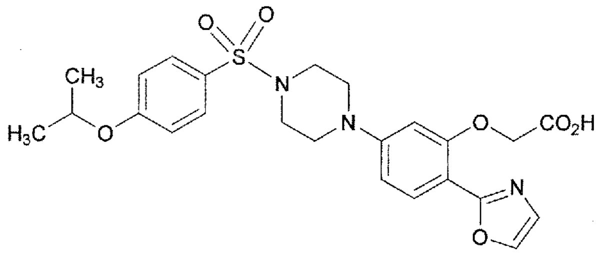

(I)

,

or a pharmaceutically acceptable salt thereof, and
(B) at least one compound selected from the group consisting of cetirizine, fexofenadine, and loratadine, or a pharmaceutically acceptable salt thereof.

Alternatively, the medicament for treating allergic rhinitis of the present invention is characterized in that the medicament is a combination product of:

(A) a compound represented by the formula (I):

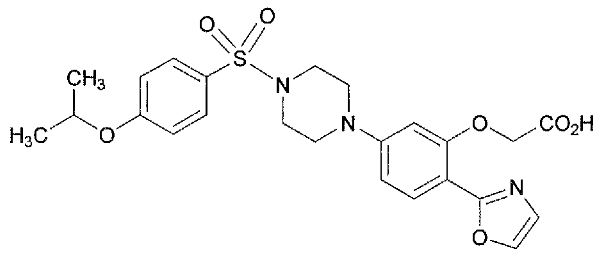

(I)

,

or a pharmaceutically acceptable salt thereof, and
(B) at least one compound selected from the group consisting of cetirizine, fexofenadine, and loratadine, or a pharmaceutically acceptable salt thereof.
Here, the medicament for treating allergic rhinitis of the present invention as used herein may be also referred to as a therapeutic agent for allergic rhinitis of the present invention.

(A) PGD2 Receptor Antagonist

The PGD2 receptor antagonist usable in the present invention is a compound represented by the formula (I), a pharmaceutically acceptable salt thereof, or a solvate thereof.
The compound represented by the formula (I) is a [2-(oxazol-2-yl)-5-(4-{4-[(propan-2-yl)oxy]phenylsulfonyl}piperazin-1-yl)phenoxy]acetic acid, and has antagonistic activity to DP receptor, which is one of the PGD2 receptors.
The compound represented by the formula (I) can be synthesized in accordance with a known method, for example, a method according to a method described in WO 2007/037187 Pamphlet or WO 2008/123349 Pamphlet.

(B) Histamine H1 Receptor Antagonist

The histamine H1 receptor antagonist usable in the present invention includes cetirizine, fexofenadine, loratadine, or a pharmaceutically acceptable salt thereof, or a solvate thereof.
Cetirizine is described in US Patent No. 4,525,358, which may form a pharmaceutically acceptable salt. The preferred pharmaceutically acceptable salt includes cetirizine hydrochloride, 2-(2-{4-[(RS)-(4-chlorophenyl)phenylmethyl]piperazin-1-yl}ethoxy)acetic acid dihydrochloride. The dose of cetirizine alone, calculated as cetirizine hydrochloride, is 10 mg, and at most 20 mg per adult per day.
Fexofenadine is described in US Patent No. 4,254,129, which may form a pharmaceutically acceptable salt. The preferred pharmaceutically acceptable salt includes fexofenadine hydrochloride and (±)-2-{4-[1-hydroxy-4-[4-(hydroxydiphenylmethyl)piperidino]butyl]phenyl}-2-methylpropanoic acid monohydrochloride. The dose of fexofenadine alone, calculated as fexofenadine hydrochloride, is 60 mg per adult per dose, twice a day.

Loratadine is described in US Patent No. 4,282,233 and includes ethyl 4-(8-chloro-5,6-dihydro-11H-benzo[5,6] cyclohepta[1,2-b]pyridin-11-ylidene)-1-piperidinecarboxylate, which may form a pharmaceutically acceptable salt. The dose of loratadine alone, calculated as loratadine, is 10 mg per adult per day.

These histamine H1 receptor antagonists may be synthesized in accordance with a known method, or commercially available products may be used.

The term "pharmaceutically acceptable salt" as used herein include basic salts including, for example, alkali metal salts such as lithium salts, sodium salts, and potassium salts; alkaline earth metal salts such as calcium salts, magnesium salts, and barium salts; transition metal salts such as zinc salts and iron salts; ammonium salts; aliphatic amine salts such as trimethylamine salts, triethylamine salts, dicyclohexylamine salts, ethanolamine salts, diethanolamine salts, triethanolamine salts, ethylenediamine salts, meglumine salts, and procaine salts; aralkylamine salts such as N,N-dibenzylethylenediamine; heterocyclic aromatic amine salts such as pyridine salts, picoline salts, quinoline salts, and isoquinoline salts; quaternary ammonium salts such as tetramethylammonium salts, tetraethylammonium salts, benzyltrimethylammonium salts, benzyltriethylammonium salts, benzyltributylammonium salts, methyltrioctylammonium salts, and tetrabutylammonium salts; and basic amino acid salts such as arginine salts and lysine salts, and the like. The acidic salts include, for example, inorganic acid salts such as hydrochlorides, sulfates, nitrates, phosphates, carbonates, hydrogencarbonates, hydrobromates, hydroiodates, and perchlorates; organic acid salts such as formates, acetates, propionates, trifluoroacetates, citrates, lactates, tartrates, oxalates, maleates, fumarates, mandelates, glutarates, malates, benzoates, phthalates, and ascorbates; sulfonates such as methanesulfonates, ethanesulfonates, isethionates, benzenesulfonates, and p-toluenesulfonates; acidic amino acids such as aspartates and glutamates; and the like.

The solvates include solvates with an organic solvent that coordinate with any number of organic solvent molecules, and hydrates coordinating with any number of water molecules. The term "solvate" as used herein means a solvate of the above compound represented by the formula (I) or a pharmaceutically acceptable salt, or a solvate of one or more compounds selected from the group consisting of cetirizine, fexofenadine, and loratadine, or a pharmaceutically acceptable salt thereof, and includes a monosolvate, a disolvate, a monohydrate, a dihydrate, and the like. monosolvate, a disolvate, a monohydrate, a dihydrate, and the like.

Here, the pharmaceutically acceptable salts and solvates can be synthesized in accordance with a known method.

The component (A) in the present invention includes the compound represented by the formula (I) mentioned above, or a sodium salt thereof, a calcium salt thereof, a magnesium salt thereof, a potassium salt thereof, and the like.

Also, the component (B) in the present invention includes, for example, cetirizine, cetirizine hydrochloride, fexofenadine, fexofenadine hydrochloride, loratadine, or a mixture thereof.

The above-mentioned combination of the component (A) and the component (B) includes combinations of a compound represented by the formula (I), a sodium salt thereof, a calcium salt thereof, a magnesium salt thereof, a potassium salt thereof, or the like and cetirizine; a compound represented by the formula (I), a sodium salt thereof, a calcium salt thereof, a magnesium salt thereof, a potassium salt thereof, or the like and cetirizine hydrochloride; a compound represented by the formula (I), a sodium salt thereof, a calcium salt thereof, a magnesium salt thereof, a potassium salt thereof, or the like and fexofenadine; a compound represented by the formula (I), a sodium salt thereof, a calcium salt thereof, a magnesium salt thereof, a potassium salt thereof, or the like and fexofenadine hydrochloride; and a compound represented by the formula (I), a sodium salt thereof, a calcium salt thereof, a magnesium salt thereof, a potassium salt thereof, or the like and loratadine. In addition, one embodiment includes a combination of a compound represented by the formula (I) and cetirizine hydrochloride.

The medicament for treating allergic rhinitis of the present invention is not particularly limited so long as the medicament contains the above component (A) and component (B) in combination, and the medicament can contain other active ingredients, within the range that would not impair the effects of the present invention. For example, the medicament can be used together with, or in the form of a combination product with leukotriene receptor antagonist (e.g., montelukast sodium, zafirlukast, pranlukast hydrate, leukotriene B4 receptor antagonist); leukotriene synthesis inhibitors (e.g., zileuton), PDE IV inhibitors (e.g., theophylline, cilomilast, roflumilast), corticosteroid (e.g., prednisolone, fluticasone, budesonide, ciclesonide), β2-agonist (e.g., salbutamol, salmeterol, formoterol, anti-IgE antibody formulation (e.g., omalizumab), histamine H1 receptor antagonist (e.g., chlorpheniramine), immunosuppressant (e.g., Protopic, cyclosporin, etc.), thromboxane A2 receptor antagonist (e.g., ramatroban), chemokine receptor (e.g., CCR-1, CCR-2, CCR-3) antagonist, other prostanoid receptor antagonist (e.g., PGD2 receptor antagonist, CRTH2 antagonist), adhesion molecule antagonist (e.g., VLA-4 antagonist), cytokine antagonist (e.g., anti-IL-4 antibody, anti-IL-3 antibody), non=steroidal anti-inflammatory agent (e.g., propionic acid derivative such as ibuprofen, ketoprofen, and naproxen; acetic acid derivative such as indomethacin and diclofenac; salicylic acid such as acetylsalicylic acid; cyclooxygenase-2 inhibitor such as celecoxib and etericoxib. Further, antitussive agent (e.g., codeine, hydrocodeine, etc.), cholesterol lowering agent (e.g., lovastatin, simvastatin, fluvastatin, rosuvastatin, etc.), anticholinergic drug (e.g., tiotropium, ipratropium,

flutropium, oxitropium, etc.) can be also used as other active ingredients. The contents of these agents are not particularly limited.

In addition, as other raw materials for formulations, the formulation may contain additives including excipients, binders, disintegrating agents, lubricants, sweeteners, corrigents, preservatives, chelating agents, antioxidants, cooling agents, coating agents, stabilizers, fluidizing agents, thickening agents, dissolution aids, thickeners, buffers, flavors, colorants, adsorbents, wetting agents, dampproof agents, antistatic agents, plasticizers, defoaming agents, surfactants, and emulsifiers. Specific examples include binders (e.g., cornstarch etc.), fillers (e.g., lactose, fine crystalline cellulose, etc.), disintegrants (e.g., starch glycolic acid sodium etc.), lubricants (e.g., magnesium stearate etc.), and the like. The contents of these agents are not particularly limited.

The medicament for treating allergic rhinitis of the present invention is not particularly limited so long as the component (A) is combined in therapy with the component (B) mentioned above, and the medicament can be prepared in accordance with a method known to one of ordinary skill in the art. Also, the shapes and sizes of the medicament are not particularly limited, and the formulations for orally administration are preferred, especially, solid formulations are more preferred. The dosage forms of the solid formulations can be exemplified by tablet (including orally fast disintegrable tablet, chewable tablet, foaming tablet, gel-like drip, etc.), troche, granule, pill, powder (including fine powder), capsule (including hard capsule and soft capsule), etc. In addition, in the preparation of these dosage forms, the medicament may be subj ected to granulation, or may be subjected to a coating treatment in accordance with a known method.

Specific examples of the medicament for treating allergic rhinitis of the present invention include a combination product containing the compound represented by the formula (I) and cetirizine or cetirizine hydrochloride; a combination of a tablet of the compound represented by the formula (I) and a tablet of cetirizine or cetirizine hydrochloride (including the kit); a combination product containing the compound represented by the formula (I) and fexofenadine or fexofenadine hydrochloride; a combination of a tablet of the compound represented by the formula (I) and a tablet of fexofenadine or fexofenadine hydrochloride (including the kit); and a combination product containing the compound represented by the formula (I) and loratadine; and a combination of a tablet of the compound represented by the formula (I) and a tablet of loratadine (including the kit).

In a case where a medicament for treating allergic rhinitis of the present invention is in the form of a tablet, the combination product can be prepared by adding intended formulation raw materials to the component (A) and the component (B) mentioned above while mixing, and introducing the mixture obtained directly to, or subsequent to granulation in accordance with a known method, into a tableting machine to mold working. Also, the tablets of each of the components can be prepared by mixing intended formulation raw materials for each of the component (A) and the component (B) mentioned above, and introducing the mixture obtained directly to, or subsequent to granulation in accordance with a known method, into a tableting machine to mold working.

The medicament for treating allergic rhinitis of the present invention has the feature that the component (A) is combined with the component (B) as active ingredients, and the use embodiments thereof include an embodiment where each of the component (A) and the component (B) that are separately prepared are used simultaneously; an embodiment where each of the component (A) and the component (B) that are separately prepared are used alone separately; an embodiment where the component (A) and the component (B) are together formulated to be used as a formulation (combination product). The present invention includes an embodiment of use as a combination product, from the viewpoint of QOL (Quality Of Life) of patients.

The dose of the component (A) and the component (B), when used as a medicament for treating allergic rhinitis, differs depending upon the administration forms, symptoms, age, body weight, sex of patients, or other drugs to be combined (if applicable), and the like, and the dose is finally rendered to the physicians' discretions. For example, in a case where the component (B) is cetirizine or a pharmaceutically acceptable salt thereof, an embodiment of orally administering 10 to 200 mg of the component (A) and 2.5 to 10 mg of the component (B), per adult per day is included. Further, an embodiment includes orally administering 50 to 100 mg of the component (A) and 5 to 10 mg of the component (B), or 10 to 100 mg of the component (A) and 5 to 10 mg of the component (B), per adult per day.

For example, in a case where the component (B) is fexofenadine or a pharmaceutically acceptable salt, an embodiment includes orally administering 10 to 200 mg of the component (A) and 30 to 120 mg of the component (B), per adult per day. Further, an embodiment includes orally administering 50 to 100 mg of the component (A) and 60 to 120 mg of the component (B), per adult per day.

For example, in a case where the component (B) is loratadine or a pharmaceutically acceptable salt, an embodiment includes orally administering 10 to 200 mg of the component (A) and 2.5 to 10 mg of the component (B), per adult per day. Further, an embodiment includes orally administering 50 to 100 mg of the component (A) and 5 to 10 mg of the component (B), per adult per day.

Here, the dose may be administered at one time or administered in divided portions.

The medicament of the present invention has excellent effects of suppressing allergic rhinitis symptoms by synergistic effects of the component (A) and the component (B), and the agent can be administered as a prophylactic agent for the prevention of rhinitis symptoms caused by allergy. Here, the term "allergic rhinitis" is not limited so long as

it is a disease showing symptoms (e.g., nasal congestion, sneezing, nasal discharge, etc.) based on the allergic inflammation in nasal mucous membranes or the like, specifically including allergic (exogenous) rhinitis, non-allergic (endogenous) rhinitis, seasonal allergic rhinitis, sinusitis, and the like. In addition, the term "synergistic effects" means a case where combinational effects of two or more kinds (usually two kinds) of agents are significantly larger than the sum of the effects for a single dose of the agents.

The present invention also provides a method for treating allergic rhinitis, including the step of administering in combination a component (A) and a component (B) in therapeutically effective amounts thereof to an individual in need of the treatment of allergic rhinitis.

The individual in need of the treatment of allergic rhinitis as used herein preferably human in need of suppressive action on allergic rhinitis symptoms, and the individual may be a pet animal.

Also, the therapeutically effective amount as used herein refers to in a case where a component (A) and a component (B) are administered in combination to the above individual, an amount that suppresses allergic rhinitis symptoms, as compared to those individual without administration. A specific effective amount is not unconditionally determined since the amount is properly set depending upon the administration forms, administration method, purpose, and individual age, body weight, symptoms and the like.

In the method for treatment of the present invention, a component (A) and a component (B) in combination may be directly administered to the above individual, or the components may be administered as a medicament such as a therapeutic agent for allergic rhinitis as mentioned above. In addition, the method of administration is not particularly limited, and the components may be administered, for example, via oral administration.

According to the method for treatment of the present invention, allergic rhinitis symptoms can be suppressed by synergistic effects of the component (A) and the component (B), and the rhinitis symptoms caused by allergy can be prevented.

## EXAMPLES

The present invention will be specifically described hereinbelow by means of the Examples, without intending to limit the scope of the present invention thereto.

### Test Example 1 Evaluation Test on Pharmacological Efficacy on Patients with Seasonal Allergic Rhinitis [Combination Case of Compound Represented by Formula (I) + Cetirizine Hydrochloride]

Four-hundred and seventy patients with seasonal allergic rhinitis were divided into four groups of a placebo group (157 patients), a group administered with a compound represented by the formula (I) (156 patients), a group administered with cetirizine hydrochloride (79 patients), a combination group (78 patients). Each of the groups was asked to take each preparation in accordance with the following medication schedule. Thereafter, each of the patients was daily evaluated on daytime nasal symptoms, e.g. sneezing attacks, nasal discharge, nasal congestion, and nasal itching, in accordance with the following evaluation criteria. The results were analyzed. The lower the nasal symptom scores, more alleviated the nasal symptoms.

Here, as patients with the seasonal allergic rhinitis, patients with a history of being diagnosed to have typical Japanese cedar pollinosis from at least two years of the last three years, the patients having a score to Japanese cedar pollen antigen of 2 or more according to the sera-specific IgE antibody titer examination were carried out during the screening period or within one year in the past were the subjects.

(Medication Schedule)

The medication was scheduled for a total of about 6 to 18 weeks, including a screening period of from 0 days to 12 weeks, a placebo introducing period of from 4 to 7 days, an ingestion period for each preparation of 4 weeks, and a post-observation period of 1 week.

(Administration Method)

The group administered with placebo (hereinafter referred to as placebo group) orally ingested twice a day, two placebo tablets of the compound represented by the formula (I) after breakfast, and one placebo tablet of cetirizine hydrochloride before bedtime, with a proper amount of water. The group administered with the compound represented by the formula (I) (hereinafter referred to as the formula (I) group) orally ingested twice a day, two tablets containing 50 mg of the compound represented by the formula (I) after breakfast (100 mg per day), and two placebo tablets of cetirizine hydrochloride before bedtime, with a proper amount of water. The group administered with cetirizine hydrochloride (hereinafter referred to as cetirizine hydrochloride group) orally ingested twice a day, two placebo tablets of the compound

represented by the formula (I) after breakfast, and one tablet containing 10 mg of cetirizine hydrochloride before bedtime (10 mg per day), with a proper amount of water. The combination group orally ingested twice a day, two tablets containing 50 mg of the compound represented by the formula (I) after breakfast, and one tablet containing 10 mg of cetirizine hydrochloride before bedtime, with a proper amount of water. Here, as a tablet containing 50 mg of the compound represented by the formula (I), a tablet produced in accordance with Formulation Example 1 was used, and as a tablet containing 10 mg of cetirizine hydrochloride, a commercially available tablet containing 10 mg of cetirizine hydrochloride (manufactured by TAKATA SEIYAKU Co., Ltd.) was used.

(Evaluation Criteria for Nasal Symptoms)

The evaluation criteria for the nasal symptoms are shown in Table 1 as follows. Here, the evaluation criteria for sneezing, nasal discharge, and nasal congestion were conducted in 5-ranks from scores 0 to 4, and the evaluation criterion for nasal itching was conducted in 4-ranks of scores 0 to 3.

[Table 1]

Table 1

| Kinds \ Level | Score 4 | Score 3 | Score 2 | Score 1 | Score 0 |
|---|---|---|---|---|---|
| Sneezing Attacks (Average Number of Attacks per Day) | 21 times or more | 20 to 11 times | 10 to 6 times | 5 to 1 time | Less than 1 time |
| Nasal Discharge (Average Number of Blows of Their Noses per Day) | 21 times or more | 20 to 11 times | 10 to 6 times | 5 to 1 time | Less than 1 time |
| Nasal Congestion | Completely stuffed throughout the day | Nasal congestion is very potent, mouth-breathing was experienced for considerable length of time of the day. | Nasal congestion is potent, mouth-breathing was sometimes experienced during the day. | Although no mouth-breathing is experienced, nasal congestion is found. | None |
| Nasal Itching | | Nose is itchy, very often rubbing over the nose or blowing the nose. | Nose is itchy, occasionally rubbing over the nose or wanting to blow the nose. | Nose is itchy, but not so bothersome. | None |

(Analysis)

As to the nasal symptoms evaluated by each patient, average scores/day at time points 1, 2, 3, and 4 weeks after the administration, and the week just before the administration (for example, at a time point of 2 weeks after the

administration refers to a one-week period from one week after the administration to the second week) were calculated, and changes since the start of the test were calculated and used in analysis. Here, in the between-group comparison distribution of the background factors, Fisher's exact test was used for nominal scale data, and Kruskal-Wallis test was used for continuous data or ordinal scale data. In both tests, the significance levels were set at 0.15 on both sides. In the analyses of nasal symptom items, the between-group comparison according to covariance analysis of an average change after two weeks from the administration, the between-group comparison according to covariance analysis of a change on a weekly basis, the between-group comparison of a change on a weekly basis using a linear model, and the between-group comparison according to chi-square test of improvement rate on a weekly basis were conducted. In all the comparisons, the significance level was set at 0.05 on both sides. In addition, as to the changes of nasal symptoms on a weekly basis, the between-group comparison according to covariance analysis for changes on a weekly basis using a linear model was conducted. Further, the between-group comparison according to chi-square test of improvement rate of each of the daytime nasal symptoms on a weekly basis was conducted. In the covariance analysis for a change on a weekly basis, in cases where data each week were not measured, the data were supplemented according to Last Observation Carried Forward (LOCF) method.

Of the above analyses, as to the score results for a total of three nasal symptom scores, i.e. sneezing, nasal discharge, and nasal congestion, and each of the four nasal symptoms, i.e. sneezing, nasal discharge, nasal congestion, and nasal itching, changes of scores after two weeks are shown in Tables 2 to 6, and changes in average, on a weekly basis or daily basis, are shown in Figures 1 to 10.

[Table 2]

Table 2

| [Change in Total of Three Nasal Symptom Scores] | | | | |
|---|---|---|---|---|
| | Formula (I) Group | Placebo Group | Combination Group | Cetirizine Hydrochloride Group |
| Mean ± SD | -0.49 ± 0.13 | -0.04 ± 0.13 | -1.64 ± 0.18 | -0.76 ± 0.18 |
| Difference with Placebo Group | -0.45* | - | -1.61*** | -0.73** |
| Difference with Cetirizine Hydrochloride Group | 0.28 | - | -0.88*** | - |
| Difference with Formula (I) Group | - | - | -1.15*** | - |
| Significance:* p<0.05, ** p<0.01, *** p<0.001 | | | | |

[Table 3]

Table 3

| [Change in Sneezing Scores] | | | | |
|---|---|---|---|---|
| | Formula (I) Group | Placebo Group | Combination Group | Cetirizine Hydrochloride Group |
| Mean ± SD | -0.20 ± 0.05 | -0.09 ± 0.05 | -0.79 ± 0.07 | -0.43 ± 0.07 |
| Difference with Placebo Group | -0.11 | - | -0.70*** | -0.34*** |
| Difference with Cetirizine Hydrochloride Group | 0.23** | - | -0.36*** | - |
| Difference with Formula (I) Group | - | - | -0.59*** | - |
| Significance:* p<0.05, ** p<0.01, *** p<0.001 | | | | |

[Table 4]

Table 4

| [Change in Nasal Discharge Scores] | | | | |
|---|---|---|---|---|
| | Formula (I) Group | Placebo Group | Combination Group | Cetirizine Hydrochloride Group |
| Mean ± SD | -0.08 ± 0.05 | 0.11 ± 0.05 | -0.45 ± 0.07 | -0.20 ± 0.07 |
| Difference with Placebo Group | -0.19** | - | -0.56*** | -0.31** |
| Difference with Cetirizine Hydrochloride Group | 0.13 | - | -0.24* | - |
| Difference with Formula (I) Group | - | - | -0.37*** | - |
| Significance:* p<0.05, ** p<0.01, *** p<0.001 | | | | |

[Table 5]

Table 5

| [Change in Nasal Congestion Scores] | | | | |
|---|---|---|---|---|
| | Formula (I) Group | Placebo Group | Combination Group | Cetirizine Hydrochloride Group |
| Mean ± SD | -0.20 ± 0.05 | -0.05 ± 0.05 | -0.40 ± 0.07 | -0.18 ± 0.07 |
| Difference with Placebo Group | -0.16* | - | -0.35*** | -0.13 |
| Difference with Cetirizine Hydrochloride Group | -0.03 | - | -0.23* | - |
| Difference with Formula (I) Group | - | - | -0.20* | - |
| Significance:* p<0.05, ** p<0.01, *** p<0.001 | | | | |

[Table 6]

| [Change in Nasal Itching Scores] | | | | |
|---|---|---|---|---|
| | Formula (I) Group | Placebo Group | Combination Group | Cetirizine Hydrochloride Group |
| Mean ± SD | -0.13 ± 0.04 | -0.07 ± 0.04 | -0.62 ± 0.06 | -0.35 ± 0.06 |
| Difference with Placebo Group | -0.06 | - | -0.55*** | -0.28** |
| Difference with Cetirizine Hydrochloride Group | 0.21** | - | -0.28** | - |
| Difference with Formula (I) Group | - | - | -0.49*** | - |
| Significance: *p<0.05, ** p<0.01, *** p<0.001 | | | | |

(Total of Three Nasal Symptoms)

From the results in Table 2, the changes of a total of three nasal symptom scores after two weeks from the administration were significantly larger in all the formula (I) group, the combination group, and the cetirizine hydrochloride group, as compared to that of the placebo group. In addition, the combination group had a between-group difference (95% confidence interval) with the formula (I) group and with the cetirizine hydrochloride group of -1.15 and -0.88, respectively, and the changes were significantly larger than all of these agent groups alone. Further, the changes of the

combination group showed synergistic effects surpassing the sum of the changes of the formula (I) group and the cetirizine hydrochloride group.

In addition, from Figure 1, changes in a total of the daytime three nasal symptom scores of the combination group were significantly large in any one time points of after 1 to 4 weeks from the administration, as compared to those of the placebo group, the formula (I) group, and the cetirizine hydrochloride group. Further, from Figure 2 the changes after the two days from the administration are nearly in a leveled-off state, so that it is suggested that the effects for the three nasal symptoms reach a substantially maximal pharmacological efficacy within two days after the administration. The phrase "a substantially maximal pharmacological efficacy" means effects of maximal actions which the agent is capable of exhibiting so as to sufficiently suppress or improve the symptoms.

Furthermore, Table 3 of Non-Patent Publication 1 describes clinical test results on seasonal allergic rhinitis for an intranasal steroid agent beclomethasone and a histamine H1 receptor antagonist loratadine. According to the publication, it can be seen that a change in scores of nasal symptoms, i.e. sneezing, nasal discharge, nasal congestion, and nasal itching, after two weeks since the start of the oral administration, per symptom from the placebo group is such that the change in a loratadine group is 0.31, i.e. a difference between 0.53 and 0.22, and that the change in a beclomethasone group is 0.48, i.e. a difference between 0.70 and 0.22. In other words, it can be seen that the intranasal steroid agent was 1.55 times effective of the histamine H1 receptor antagonist. On the other hand, it is considered that in Table 2 of Test Example mentioned above, the change in the cetirizine group, a histamine H1 receptor antagonist, is 0.73, so that a change per symptom is considered to be 0.24. Similarly, the change of the combination group is 1.61, so that a change per symptom is considered to be 0.54. In other words, it can be seen that the effects of the combination administration was 2.25 times those of the administration of the histamine H1 receptor antagonist.

It can be said from the above that the effects of the intranasal steroid agent and the combination effects of the compound represented by the formula (1) and cetirizine hydrochloride are nearly of the same level. By a combined use with an orally administered agent, it is quite surprising to exhibit the same level, or even higher by the combined use with the orally administered agent.

(Sneezing)

From the results of Table 3, the changes of sneezing attack scores of the combination group were significantly larger in the reduced level, than all those of the placebo group, the formula (I) group, and the cetirizine hydrochloride group. Further, the changes of the combination group showed synergistic effects surpassing the sum of the changes of the formula (I) group and the cetirizine hydrochloride group.

In addition, from Figure 3, changes in sneezing attack scores of the combination group were significantly large in all time points of after 1 to 4 weeks from the administration, as compared to all those of the placebo group, the formula (I) group, and the cetirizine hydrochloride group. Further, from Figure 4 the changes after the two or three days from the administration are nearly in a leveled-off state, so that it is suggested that the agent has immediate efficacy to the effects against sneezing.

(Nasal Discharge)

From the results in Table 4, the changes of nasal discharge scores of the combination group were significantly larger than all those of the placebo group, the formula (I) group, and the cetirizine hydrochloride group. Further, the changes of the combination group showed synergistic effects surpassing the sum of the changes of the formula (I) group and the cetirizine hydrochloride group.

In addition, from Figure 5, changes in nasal discharge scores of the combination group were significantly larger in all time points of after 1 to 4 weeks from the administration, than all those of the placebo group, the formula (I) group, and the cetirizine hydrochloride group, so that a significant difference was found, as compared to the placebo group and the formula (I) group after 1 to 4 weeks from the administration, and the cetirizine hydrochloride group after 1 and 2 weeks from the administration. Further, from Figure 6 the changes after the two days from the administration are nearly in a leveled-off state, so that it is suggested that the agent has immediate efficacy to the suppressive effects against nasal discharge.

(Nasal Congestion)

From the results of Table 5, the changes in nasal congestion scores of the combination group were significantly larger than all those of the placebo group, the formula (I) group, and the cetirizine hydrochloride group.

In addition, from Figure 7, changes in nasal congestion scores of the combination group were larger in all time points of after 1 to 4 weeks from the administration than those of the placebo group, the formula (I) group, and the cetirizine hydrochloride group, so that a significant difference was found, as compared to the placebo group after 1 to 4 weeks from the administration and the formula (I) group after 3 and 4 weeks from the administration, and the cetirizine hydrochloride group after 2 weeks from the administration.

(Nasal Itching)

From the results of Table 6, the changes in nasal itching scores of the combination group were significantly larger than all those of the placebo group, the formula (I) group, and the cetirizine hydrochloride group. Further, the changes of the combination group showed synergistic effects surpassing the sum of the changes of the formula (I) group and the cetirizine hydrochloride group.
In addition, from Figure 9, changes in nasal itching scores of the combination group were larger in all time points of after 1 to 4 weeks from the administration than those of the placebo group, the formula (I) group, and the cetirizine hydrochloride group, so that a significant difference was found, as compared to the placebo group, the formula (I) group, and the cetirizine hydrochloride group, in all time points of after 1 to 4 weeks from the administration.
It can be seen from these findings that the combination group has significant large changes in all the symptom scores as compared to the placebo group, and that a total of many of the symptom scores including the daytime three nasal symptoms also has significant large changes as compared to each of the compound represented by the formula (I) or cetirizine hydrochloride agent alone, so that synergistic effects are obtained.

<u>Test Example 2 Evaluation Test 1 for Pharmacological Effects of Guinea Pig Rhinitis Model as Subjects [Combination Case of Compound Represented by Formula (I) + Fexofenadine Hydrochloride]</u>

Guinea pig rhinitis models were generated by causing rhinitis-inflammation in 5 to 6 week-old male Harley guinea pigs in the following manner. Concretely, on day 0, the guinea pigs were intraperitoneally administered with cyclophosphamide diluted with physiological saline at 30 mg/kg. Two days later (on day 2), a mixed solution of aluminum hydroxide gel (10 mg), ovalbumin (OVA) (1 mg) and physiological saline was intraperitoneally administered to establish active sensitization. Thereafter, on day 9, a 1% OVA solution dissolved in physiological saline was intranasally challenged by 20 μL each to both the nostrils. Further, on day 16, a 2% OVA solution dissolved in physiological saline was intranasally challenged by 10 μL each to both the nostrils, thereby generating guinea pig rhinitis models that caused rhinitis-inflammation. Here, a control group that serves as a control was intranasally challenged by physiological saline in place of intranasally challenging by the OVA solution.
The guinea pig rhinitis models obtained were divided into the groups shown in the following Table 7. Specifically, prepared were the following:

Group 1: Control Group (a group administered with a medium alone to a guinea pig not causing rhinitis);
Group 2: Vehicle Group (a group administered with a medium alone to a rhinitis model);
Group 3: a group administered with a compound represented by the formula (I) at 1 mg/kg;
Group 4: a group administered with a compound represented by the formula (I) at 10 mg/kg;
Group 5: a group administered with fexofenadine hydrochloride (Fex) at 20 mg/kg;
Group 6: a group administered with a compound represented by the formula (I) at 1 mg/kg and Fex at 20 mg/kg; and
Group 7: a group administered with a compound represented by the formula (I) at 10 mg/kg and Fex at 20 mg/kg.

Here, as the compound represented by the formula (I), a compound synthesized in accordance with a method described in WO 2007/037187 Pamphlet was used, and as the fexofenadine hydrochloride, the compound manufactured by TOKYO CHEMICAL INDUSTRY CO., LTD. was used.
Next, on day 23, a test substance listed in Table 7 was orally administered using a 0.5% methylcellulose (MC) solution as a medium, and one hour thereafter a 2% OVA solution dissolved in physiological saline was intranasally challenged by 10 μL each to both the nostrils (Group 1 was intranasally challenged by physiological saline in place of intranasal challenge by the OVA solution), and the nasal airway resistance was evaluated and analyzed. The evaluation of the nasal airway resistance was carried out by calculating the nasal airway resistance from a change of each flow signal of nose part and chest part of the animal under non-invasive consciousness with a respiratory function measurement apparatus (Pulmos-I, manufactured by M. I. P. S.) according to Double-chamber Plethysmograph method before the intranasal challenge and 20 minutes after the intranasal challenge by the OVA solution. Here, nasal airway resistance (nRaw) is an average of specific airway resistance (sRaw) of 200 times or more breathing, and the analyzed values used in the comparisons of pharmacological efficacy are expressed as a percent change based on the nasal airway resistance measured before the intranasal challenge (100%) (IAR: immediately airway response). In addition, the between-group comparison was carried out according to one-sided t test, and the suppression percentage to Group 2 (Vehicle Group) was calculated by the following formula:

$$\text{Suppression Percentage (\%)} = (A-B)/(A-C) \times 100$$

wherein A: a proportion of nasal airway resistance at 20 minutes from the intranasal challenge to the nasal airway resistance before the intranasal challenge in Group 2 (%):

B: a proportion of nasal airway resistance at 20 minutes from the intranasal challenge to the nasal airway resistance before the intranasal challenge in a group administered with a test substance (%); and

C: a proportion of nasal airway resistance at 20 minutes from the intranasal challenge to the nasal airway resistance before the intranasal challenge in Group 1 (%).

The results are shown in Figure 11.

[Table 7]

Table 7

| Group | Test Substance | Compound Represented by Formula (I) (Formula (I)) (mg/kg) | Fexofenadine Hydrochloride (Fex) (mg/kg) | Intranasal Challenge | Number/ Group |
|---|---|---|---|---|---|
| 1 | 0.5% MC | - | - | Saline | 20 |
| 2 | 0.5% MC | - | - | OVA | 17 |
| 3 | Formula (I) | 1 | - | OVA | 17 |
| 4 | Formula (I) | 10 | - | OVA | 17 |
| 5 | Fex | - | 20 | OVA | 17 |
| 6 | Formula (I)+Fex | 1 | 20 | OVA | 16 |
| 7 | Formula (I)+Fex | 10 | 20 | OVA | 16 |

From Figure 11, the combination administration group (Group 6) of the compound represented by the formula (I) (1 mg/kg) and fexofenadine hydrochloride (20 mg/kg) showed 55% suppression, which showed a more potent suppression than the sum of suppression percentages of the group with the single dose of the compound represented by the formula (I) at 1 mg/kg (Group 3) and the group with the single dose of fexofenadine hydrochloride (Group 5) which was 36% (the sum of 21% and 15%). Further, the combination administration group (Group 7) with the compound represented by the formula (I) at 10 mg/kg also showed 67% suppression, and similarly showing more potent suppression than the sum of the suppression percentage of the group with the single dose of the compound represented by the formula (I) at 10 mg/kg (Group 4) and the suppression percentage of the group with the single dose of fexofenadine hydrochloride (Group 5), which was 62% (the sum of 47% and 15%).

It was suggested from the above results that the combined use of the compound represented by the formula (I), a PGD2 receptor antagonist, and fexofenadine hydrochloride, a histamine H1 receptor antagonist had increased efficacy than each of single dose therapy against the allergic rhinitis treatment.

<u>Test Example 3 Evaluation Test 2 for Pharmacological Effects of Guinea Pig Rhinitis Model as Subjects [Combination Case of Compound Represented by Formula (I) + Loratadine]</u>

Guinea pig rhinitis models were generated in the same manner as in Test Example 2 using 5- to 6-week old male Hartley guinea pigs. Also, Control Group used as a control was generated in the same manner as in

Test Example 2.

The guinea pig rhinitis models obtained were divided into the groups shown in the following Table 8. Specifically, prepared were the following:

Group 1: Control Group (a group administered with a medium alone to a guinea pig not causing rhinitis);
Group 2: Vehicle Group (a group administered with a medium alone to a rhinitis model);
Group 3: a group administered with a compound represented by the formula (I) at 1 mg/kg;
Group 4: a group administered with a compound represented by the formula (I) at 10 mg/kg;
Group 5: a group administered with loratadine (Lora) at 3 mg/kg;

Group 6: a group administered with a compound represented by the formula (I) at 1 mg/kg and Lora at 3 mg/kg; and
Group 7: a group administered with a compound represented by the formula (I) at 10 mg/kg and Lora at 3 mg/kg.

Here, as the compound represented by the formula (I), a compound synthesized in accordance with a method described in WO 2007/037187 Pamphlet was used, and as the loratadine, a loratadine reagent manufactured by TOKYO CHEMICAL INDUSTRY CO., LTD. was used.

Next, on day 23, a test substance listed in Table 8 was orally administered using a 0.5% methylcellulose (MC) solution as a medium, and one hour thereafter a 2% OVA solution dissolved in physiological saline was intranasally challenged by 10 μL each to both the nostrils (Group 1 was intranasally challenged by physiological saline in place of intranasal challenge by the OVA solution), and the nasal airway resistance was evaluated and analyzed. The evaluation and analysis of the nasal airway resistance were carried out in the same manner as in Test Example 2. The results are shown in Figure 12.

[Table 8]

Table 8

| Group | Test Substance | Compound Represented by Formula (I) (Formula (I)) (mg/kg) | Loratadine (Lora) (mg/kg) | Intranasal Challenge | Number/ Group |
|---|---|---|---|---|---|
| 1 | 0.5% MC | - | - | Saline | 15 |
| 2 | 0.5% MC | - | - | OVA | 16 |
| 3 | Formula (I) | 1 | - | OVA | 15 |
| 4 | Formula (I) | 10 | - | OVA | 15 |
| 5 | Lora | - | 3 | OVA | 16 |
| 6 | Formula (I)+ Lora | 1 | 3 | OVA | 16 |
| 7 | Formula (I)+ Lora | 10 | 3 | OVA | 16 |

From Figure 12, the combination administration group (Group 6) of the compound represented by the formula (I) (1 mg/kg) and loratadine (3 mg/kg) showed 66% suppression, and further, the combination administration group (Group 7) with the compound represented by the formula (I) at 10 mg/kg showed 80% suppression.

It was suggested from the above results that the combined use of the compound represented by the formula (I), a PGD2 receptor antagonist, and loratadine, a histamine H1 receptor antagonist had increased efficacy than each of single dose therapy against the allergic rhinitis treatment.

Test Example 4 Evaluation Test 3 for Pharmacological Effects of Guinea Pig Rhinitis Model Induced with PGD2 and Histamine as Subjects [Combination Case of Compound Represented by Formula (I) + Cetirizine Hydrochloride]

The pharmacological efficacy evaluation was made using 9 to 12 week-old male Harley guinea pigs in the following manner.

The guinea pigs were divided into the groups shown in the following Table 9. Specifically, the guinea pigs were divided into the following groups:

Group 1: Control Group (a group administered with a medium alone to a guinea pig);
Group 2: Vehicle Group (a group administered with a medium alone to a rhinitis model induced with PGD2 and histamine);
Group 3: a group administered with a compound represented by the formula (I) at 10 mg/kg;
Group 4: a group administered with cetirizine hydrochloride (Cet) at 10 mg/kg; and
Group 5: a group administered with a compound represented by the formula (I) at 10 mg/kg and Cet at 10 mg/kg,

to conduct the test. Here, as the compound represented by the formula (I), a compound synthesized in accordance with a method described in WO 2007/037187 Pamphlet was used, and as the cetirizine hydrochloride, the compound manufactured by Wako Pure Chemicals Industries, Ltd. was used.

The guinea pigs were orally administered with a test substance using a 0.5% methylcellulose (MC) solution as

a medium, and one hour thereafter a solution of PGD2 (20 mg/mL) and histamine (2 mg/mL) dissolved in a 20% ethanol containing physiological saline was intranasally challenged by 10 μL each to both the nostrils, and about 10 minutes thereafter, the nasal airway resistance of the guinea pigs was measured.

The nasal airway resistance was calculated in the same manner as in Test Example 2. Here, nasal airway resistance (nRaw) is an average of specific airway resistance (sRaw) of 100 times or more breathing, and the analyzed values used in the comparisons of pharmacological efficacy are expressed as a percent change based on the nasal airway resistance measured before the intranasal challenge (100%). In addition, the between-group comparison and the suppression percentage to Group 2 (Vehicle Group) were conducted and calculated in the same manner as in Test Example 2. The results are shown in Figure 13.

[Table 9]

Table 9

| Group | Test Substance | Compound Represented by Formula (I) (Formula (I)) (mg/kg) | Cetirizine Hydrochloride (Cet) (mg/kg) | Intranasal Challenge | Number/ Group |
|---|---|---|---|---|---|
| 1 | 0.5% MC | - | - | Saline | 9 |
| 2 | 0.5% MC | - | - | PGD2 + Histamine | 9 |
| 3 | Formula (I) | 10 | - | PGD2 + Histamine | 9 |
| 4 | Cet | - | 10 | PGD2 + Histamine | 9 |
| 5 | Formula (I) + Cet | 10 | 10 | PGD2 + Histamine | 9 |

It can be seen from Figure 13 that the combination administration group (Group 5) of the compound represented by the formula (I) (10 mg/kg) and cetirizine hydrochloride (10 mg/kg) showed 97% suppression. Since the sum of the suppression percentage of the group with the single dose of the compound represented by the formula (I) at 10 mg/kg (Group 3) (32%) and the suppression percentage of the group with the single dose of cetirizine hydrochloride (Group 4) (20%) was 52%, it can be seen that the combination administration group shows suppression equal to or greater than the additive effect.

It was suggested from the above results that the combined use of the compound represented by the formula (I), a PGD2 receptor antagonist, and cetirizine hydrochloride, a histamine H1 receptor antagonist had increased efficacy than each of single dose therapy against the allergic rhinitis treatment.

Formulation Examples

The following Formulation Examples 1 to 4 are given solely for the purposes of illustration and are not intending to limit the present invention in any manner.

Formulation Example 1

A tablet containing 50 mg of a compound represented by the formula (I) was prepared as follows:

A compound represented by the formula (I), D-mannitol, and croscarmellose sodium were mixed. The powders obtained were granulated with an aqueous solution of hydroxypropyl cellulose, and a granulated product was pulverized. The granules thus obtained were dried, and croscarmellose sodium, water-containing silicon dioxide, and magnesium stearate were added thereto, while mixing, and thereafter the mixture was compressed with a tableting machine to give an uncoated tablet. The uncoated tablet was coated with a coating solution containing hypromellose, titanium oxide, triethyl citrate, and talc to give a tablet having the mass of 105.2 mg.

[Table 10]

Table 10

| Ingredients | Amount (mg) |
|---|---|
| Compound Represented by Formula (I) | 50.0 |
| D-Mannitol | 40.0 |
| Croscarmellose Sodium | 5.0 |
| Hydroxypropyl Cellulose | 1.0 |
| Water-Containing Silicon Dioxide | 2.0 |
| Magnesium Stearate | 2.0 |
| Hypromellose | 3.42 |
| Titanium Oxide | 1.05 |
| Triethyl Citrate | 0.39 |
| Talc | 0.34 |
| Total | 105.2 |

Formulation Example 2

A tablet containing 10 mg of a compound represented by the formula (I) was prepared as follows:

A compound represented by the formula (I), D-mannitol, and croscarmellose sodium were mixed. The powders obtained were granulated with an aqueous solution of hydroxypropyl cellulose, and a granulated product was pulverized. The granules thus obtained were dried, and croscarmellose sodium and magnesium stearate were added thereto, while mixing, and thereafter the mixture was compressed with a tableting machine to give an uncoated tablet. The uncoated tablet was coated with a coating solution containing hypromellose, titanium oxide, triethyl citrate, and talc to give a tablet having the mass of 104 mg.

[Table 11]

Table 11

| Ingredients | Amount (mg) |
|---|---|
| Compound Represented by Formula (I) | 10.0 |
| D-Mannitol | 82.0 |
| Croscarmellose Sodium | 5.0 |
| Hydroxypropyl Cellulose | 2.0 |
| Magnesium Stearate | 1.0 |
| Hypromellose | 2.63 |
| Titanium Oxide | 0.81 |
| Triethyl Citrate | 0.30 |
| Talc | 0.26 |
| Total | 104.0 |

Formulation Example 3

A tablet containing 100 mg of a compound represented by the formula (I) and 10 mg of cetirizine hydrochloride is prepared as follows:

| | |
|---|---|
| Compound Represented by Formula (I) | 100 mg |
| Cetirizine Hydrochloride | 10 mg |
| Starch | 45 mg |
| Microcrystalline Cellulose | 35 mg |
| Polyvinyl Pyrrolidone (10% solution in water) | 4 mg |
| Sodium Carboxymethyl Starch | 4.5 mg |
| Magnesium Stearate | 0.5 mg |

(continued)

| | |
|---|---|
| Talc | 1 mg |
| Total | 200 mg |

The compound represented by the formula (I), cetirizine hydrochloride, starch, and microcrystalline cellulose are sieved with No. 45 mesh U.S. sieve, and the ingredients are sufficiently mixed. The powder obtained is mixed with an aqueous solution containing polyvinyl pyrrolidone, and the mixture is then sieved through a No. 14 mesh U.S. sieve. The granules thus obtained are dried at 50°C, and dried granules are sieved through a No. 18 mesh U.S. sieve. Sodium carboxymethyl starch, magnesium stearate, and talc, previously sieved through a No. 60 mesh U.S. sieve, are added to the granules, and the ingredients are mixed, and then compressed with a tableting machine to give a tablet each having the weight of 200 mg.

Formulation Example 4

A capsule containing 100 mg of a compound represented by the formula (I) and 10 mg of cetirizine hydrochloride is prepared as follows:

| | |
|---|---|
| Compound Represented by Formula (I) | 100 mg |
| Cetirizine Hydrochloride | 10 mg |
| Starch | 59 mg |
| Microcrystalline Cellulose | 59 mg |
| Magnesium Stearate | 2 mg |
| Total | 230 mg |

The compound represented by the formula (I), cetirizine hydrochloride, starch, microcrystalline cellulose, and magnesium stearate are mixed, and sieved through a No. 45 mesh U.S. sieve, and the powder is filled into a hard gelatin capsule in an amount of 230 mg each.

INDUSTRIAL APPLICABILITY

Since the effects of the medicament for treating allergic rhinitis of the present invention surpass the sum of the effects of single dose of each of the agents against the allergic rhinitis, the medicament is useful as a medicament for treating allergic rhinitis having potent effects. In addition, the medicament for treating allergic rhinitis of the present invention exhibits excellent effects of exhibiting substantially maximum pharmacological efficacy within two days from the start of administration. Further, the medicament for treating allergic rhinitis of the present invention is also useful as an orally administered medicament for treating allergic rhinitis showing the same level of pharmacological efficacy as the intranasal steroid agent having the strongest pharmacological efficacy.

## Claims

1. A medicament for treating allergic rhinitis, **characterized in that** a compound represented by the formula (I):

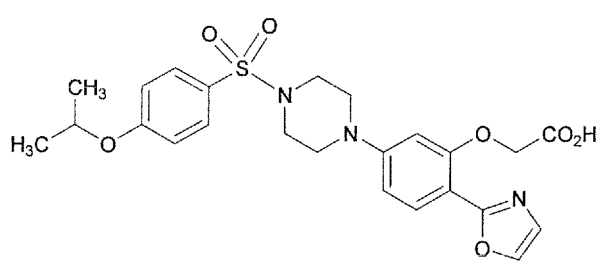

(I)

,

or a pharmaceutically acceptable salt thereof is combined with at least one compound selected from the group consisting of cetirizine, fexofenadine, and loratadine, or a pharmaceutically acceptable salt thereof.

2. The medicament according to claim 1, wherein the allergic rhinitis is perennial allergic rhinitis or seasonal allergic rhinitis.

3. The medicament according to claim 1 or 2, wherein the medicament reaches substantially maximum pharmacological efficacy for nasal congestion, sneezing, or nasal discharge, within two days since the start of administration.

4. The medicament according to any one of claims 1 to 3, wherein the medicament is an orally administered agent.

5. A combination product for treating allergic rhinitis, comprising a compound represented by the formula (I):

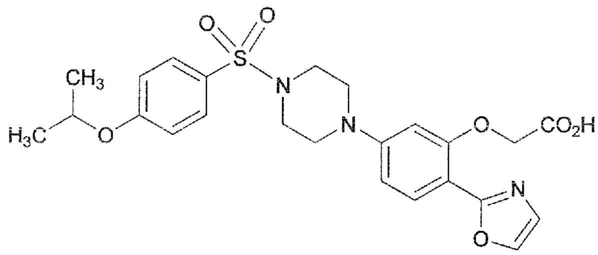

(I)

,

or a pharmaceutically acceptable salt thereof, and
at least one compound selected from the group consisting of cetirizine, fexofenadine, and loratadine, or a pharmaceutically acceptable salt thereof.

6. The combination product according to claim 5, wherein the allergic rhinitis is perennial allergic rhinitis or seasonal allergic rhinitis.

7. The combination product according to claim 5 or 6, wherein the combination product reaches substantially maximum pharmacological efficacy for nasal congestion, sneezing, or nasal discharge, within two days since the start of administration.

8. The combination product according to any one of claims 5 to 7, wherein the combination product is an orally administered agent.

9. A method for treating allergic rhinitis, comprising the steps of administering in combination a compound represented by the formula (I):

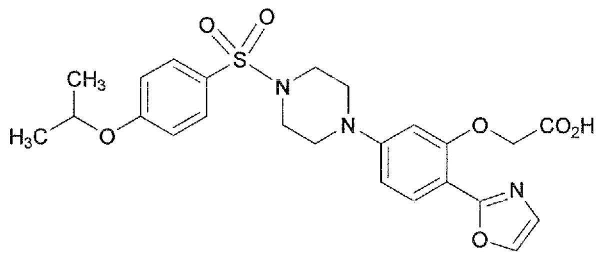

(I)

,

or a pharmaceutically acceptable salt thereof, and
at least one compound selected from the group consisting of cetirizine, fexofenadine, and loratadine, or a pharmaceutically acceptable salt thereof in a therapeutically effective amount thereof to an individual in need of treatment for allergic rhinitis.

10. The method of treatment according to claim 9, wherein the allergic rhinitis is perennial allergic rhinitis or seasonal allergic rhinitis.

11. A pharmaceutical composition for use in the treatment of allergic rhinitis, comprising a compound represented by the formula (I):

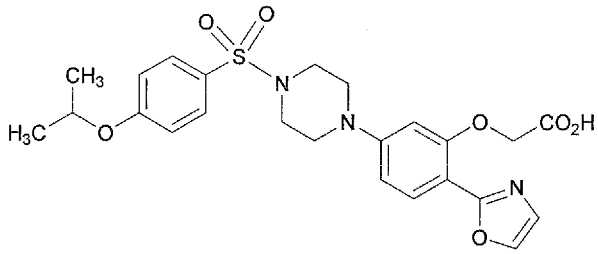

(I)

,

or a pharmaceutically acceptable salt thereof, in combination with at least one compound selected from the group consisting of cetirizine, fexofenadine, and loratadine, or a pharmaceutically acceptable salt thereof.

**12.** The pharmaceutical composition according to claim 11, wherein the allergic rhinitis is perennial allergic rhinitis or seasonal allergic rhinitis.

**13.** A compound represented by the formula (I):

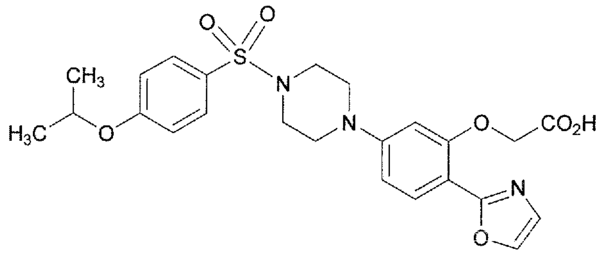

(I)

,

or a pharmaceutically acceptable salt thereof, for use in the treatment of allergic rhinitis, wherein the compound or a pharmaceutically acceptable salt thereof is used together with at least one compound selected from the group consisting of cetirizine, fexofenadine, and loratadine, or a pharmaceutically acceptable salt thereof.

**14.** The compound or a pharmaceutically acceptable salt thereof according to claim 13, wherein the allergic rhinitis is perennial allergic rhinitis or seasonal allergic rhinitis.

**15.** A pharmaceutical formulation for use in the treatment of allergic rhinitis comprising, in a single dosage form or separate dosage forms,

(A) a compound represented by the formula (I):

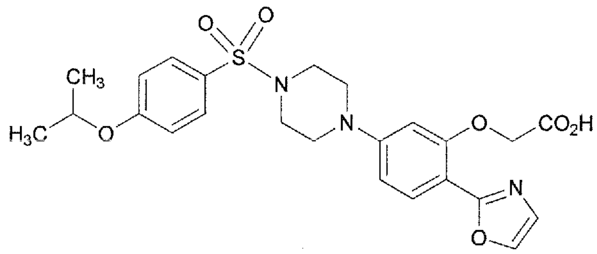

(I)

,

or a pharmaceutically acceptable salt thereof, and
(B) at least one compound selected from the group consisting of cetirizine, fexofenadine, and loratadine, or a pharmaceutically acceptable salt thereof.

[Figure 1]

FIG. 1

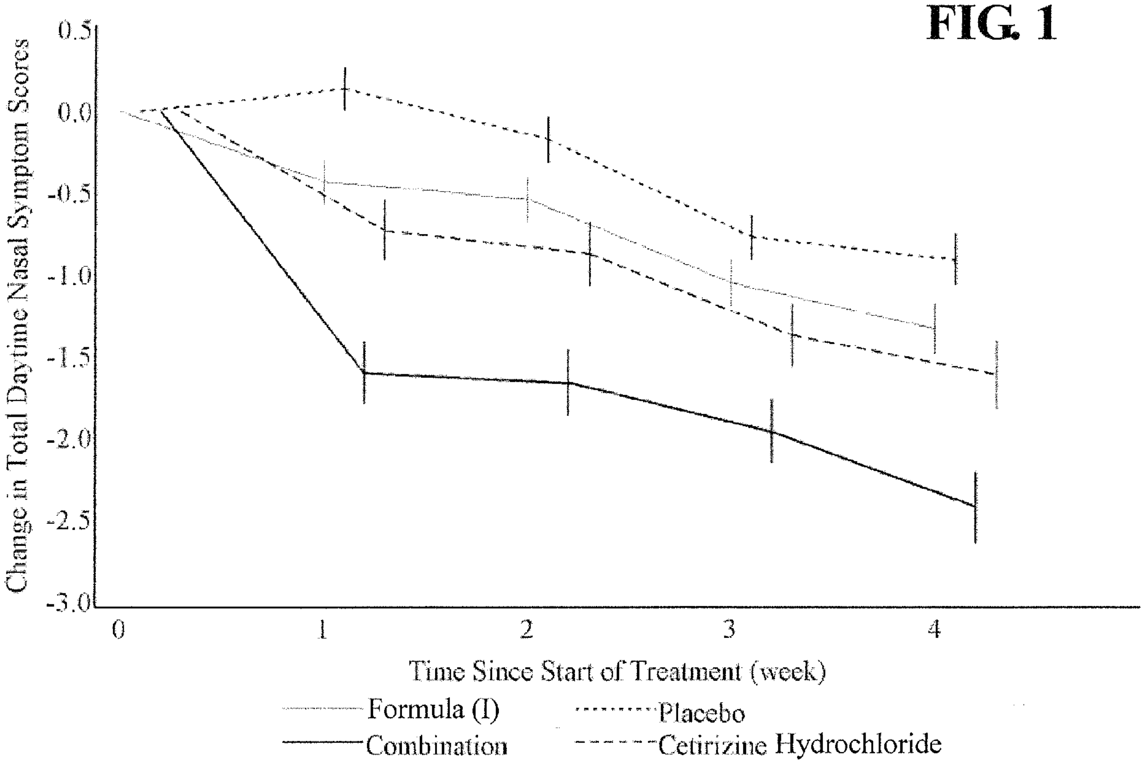


[Figure 2]

FIG. 2

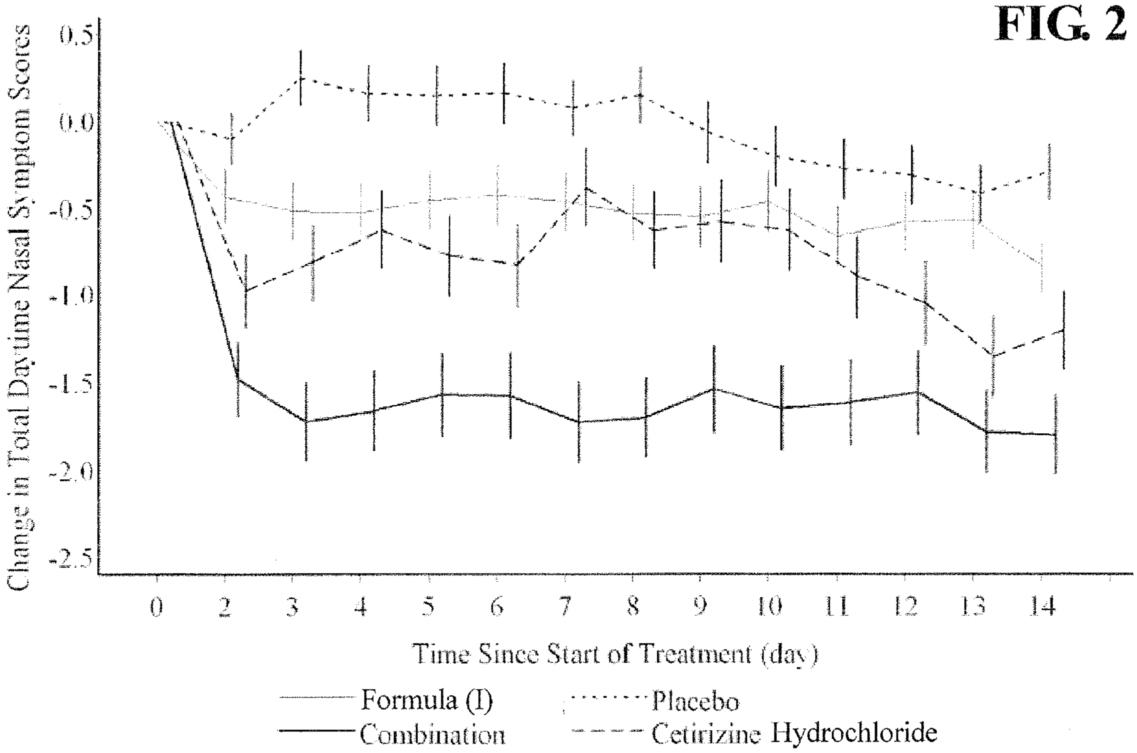

[Figure 3]

FIG. 3

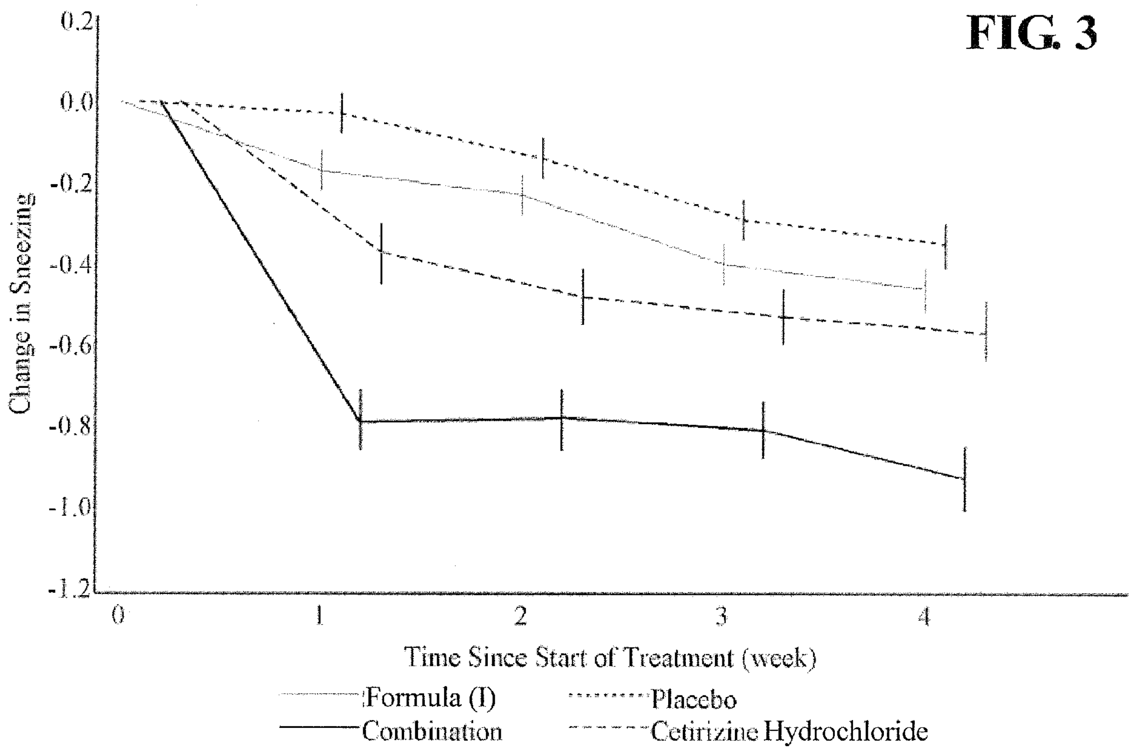


[Figure 4]

FIG. 4

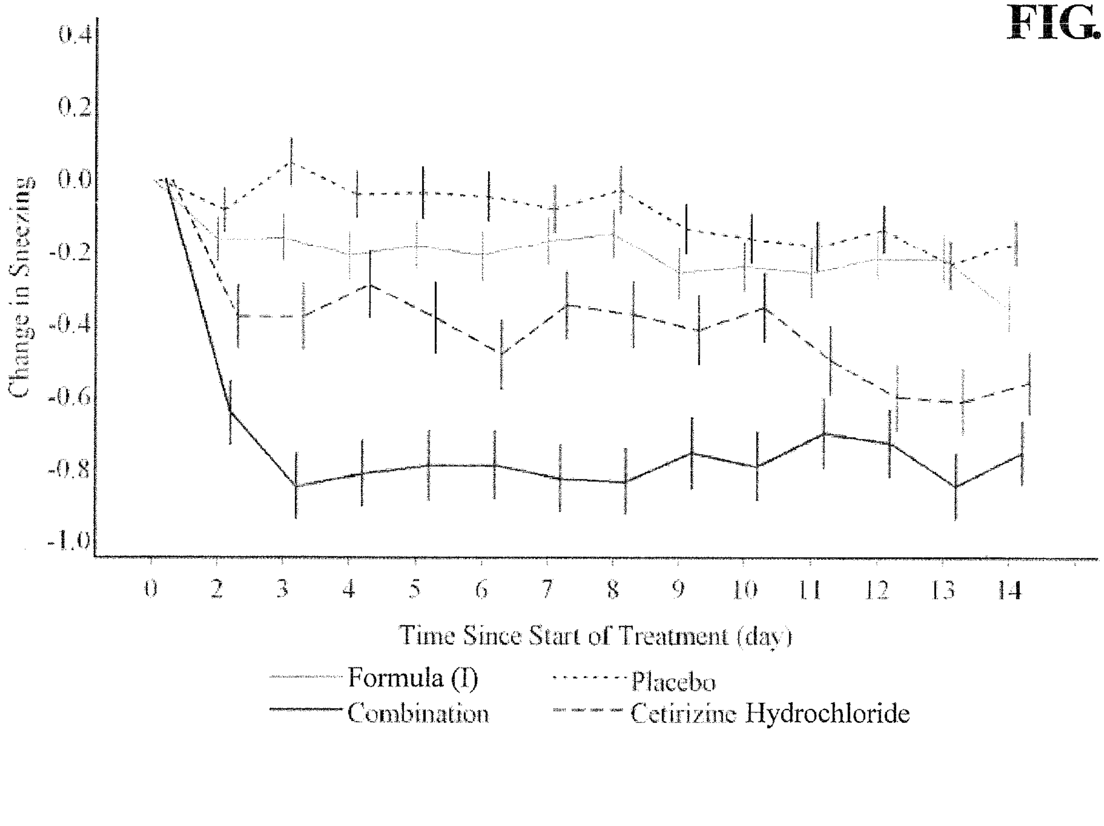

[Figure 5]

FIG. 5

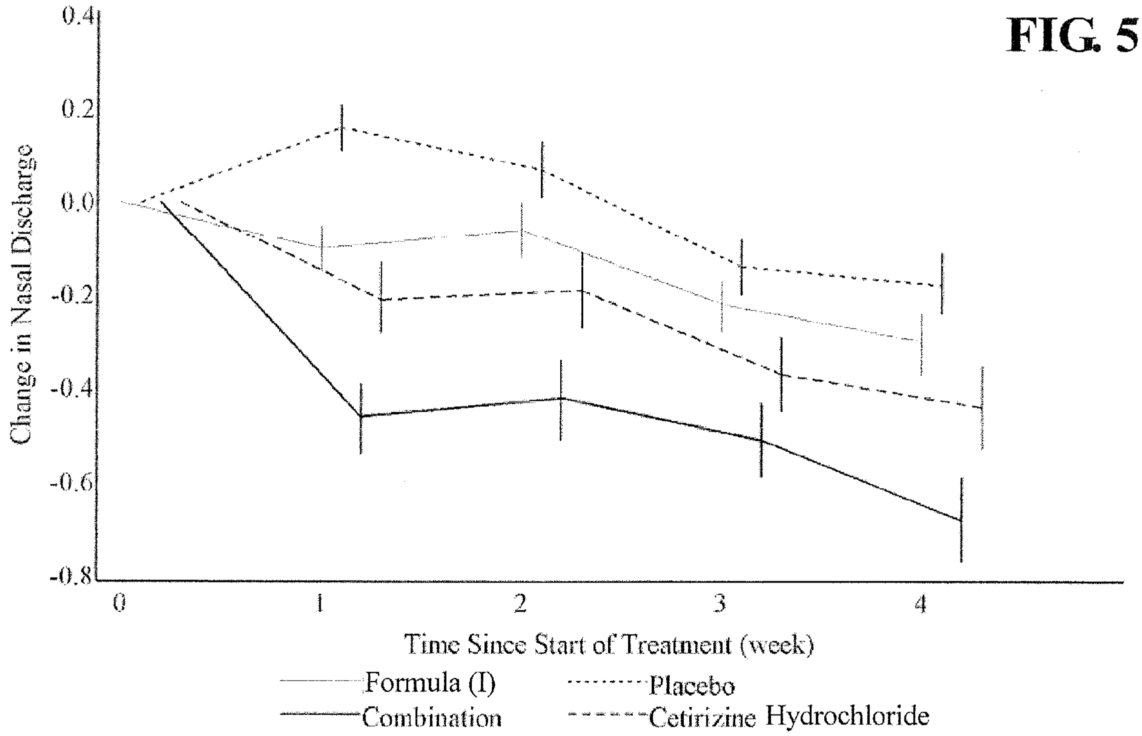


[Figure 6]

FIG. 6

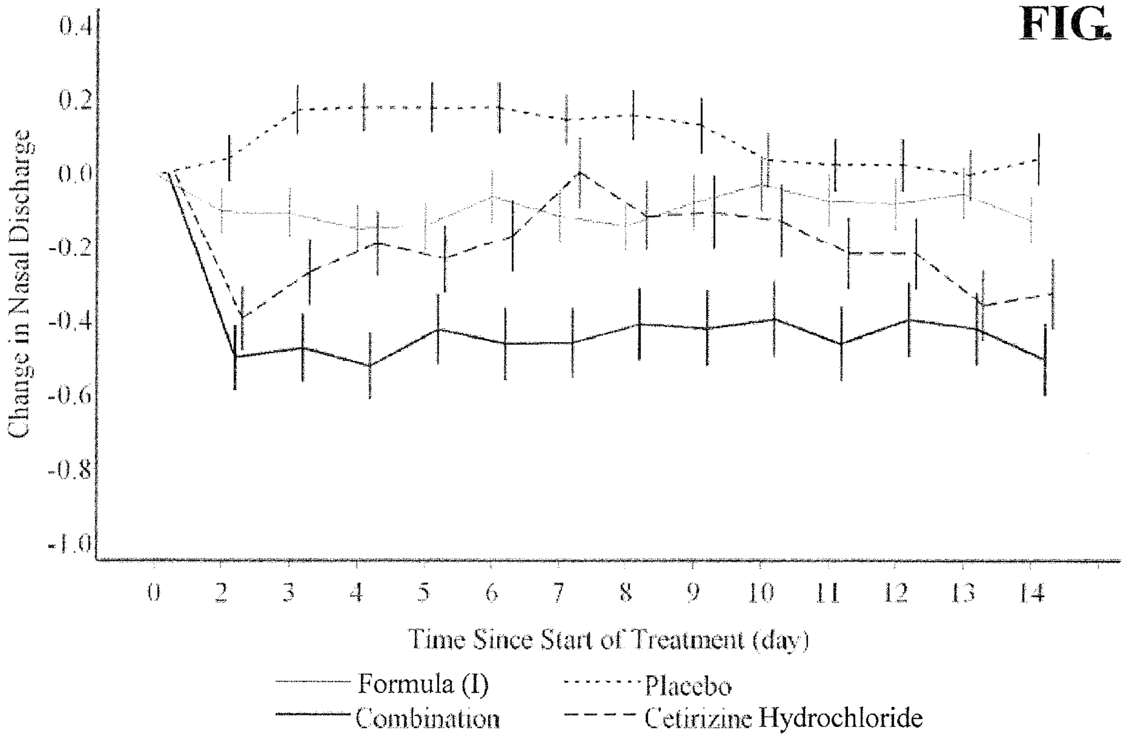

[Figure 7]

FIG. 7

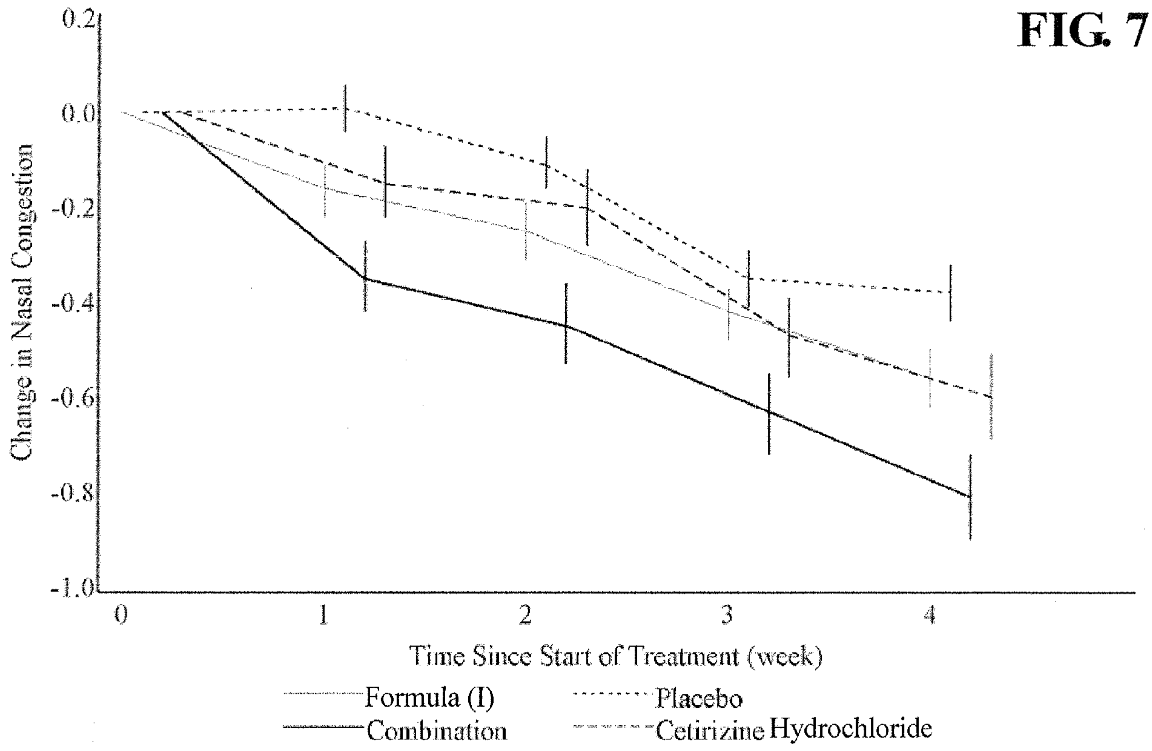


[Figure 8]

FIG. 8

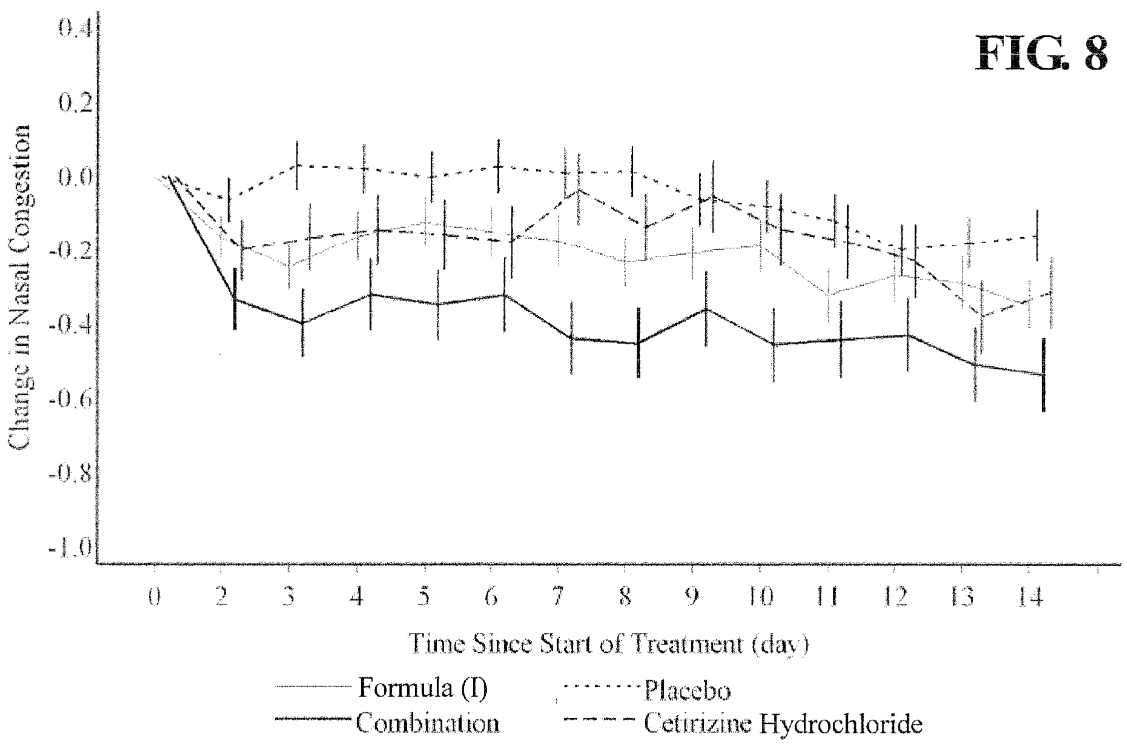

[Figure 9]

FIG. 9

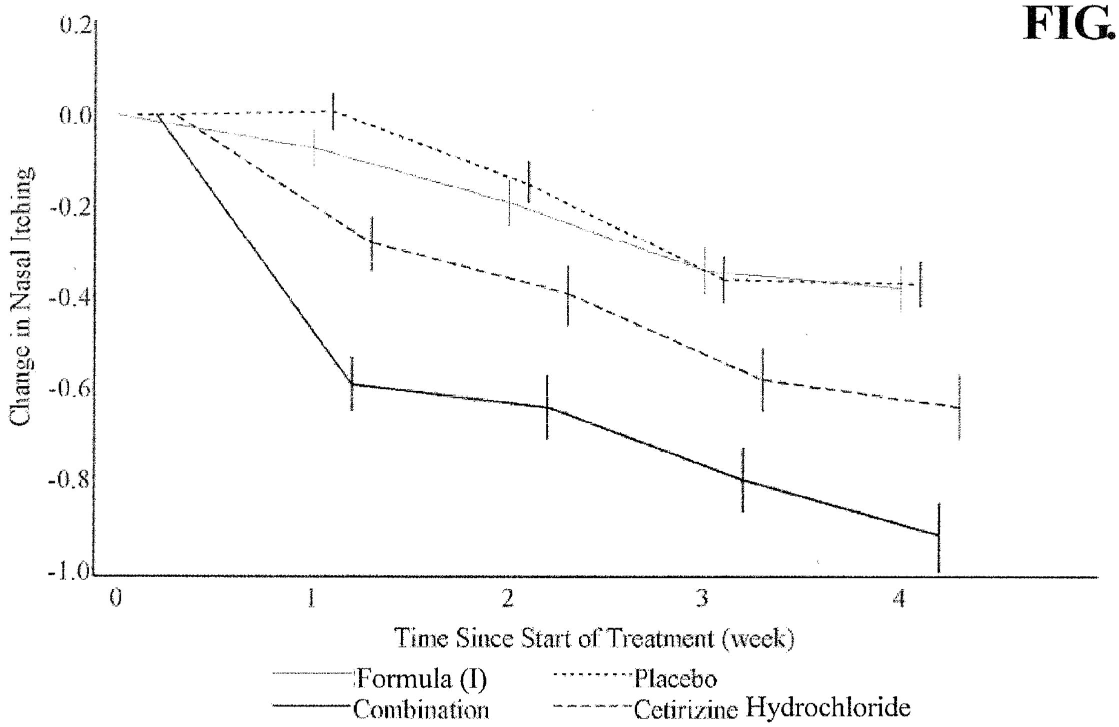


[Figure 10]

FIG. 10

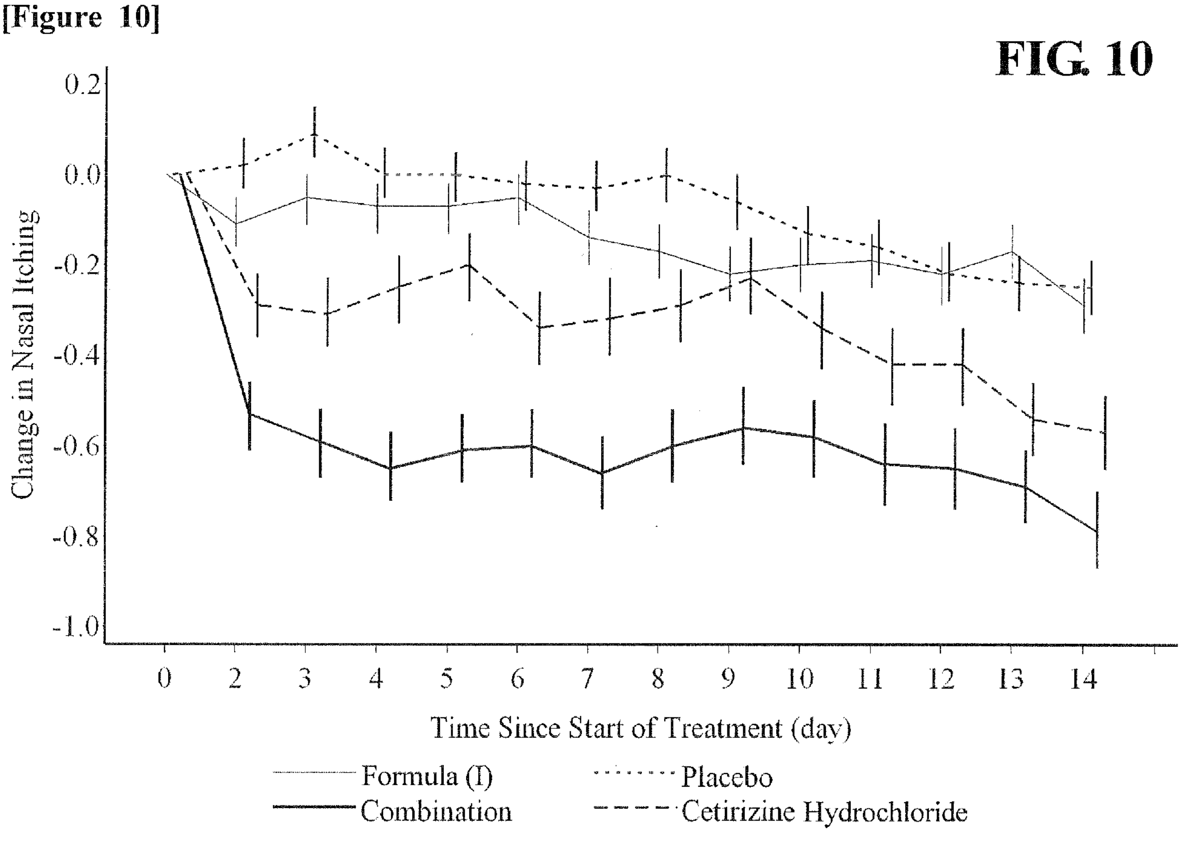

[Figure 11]

FIG. 11

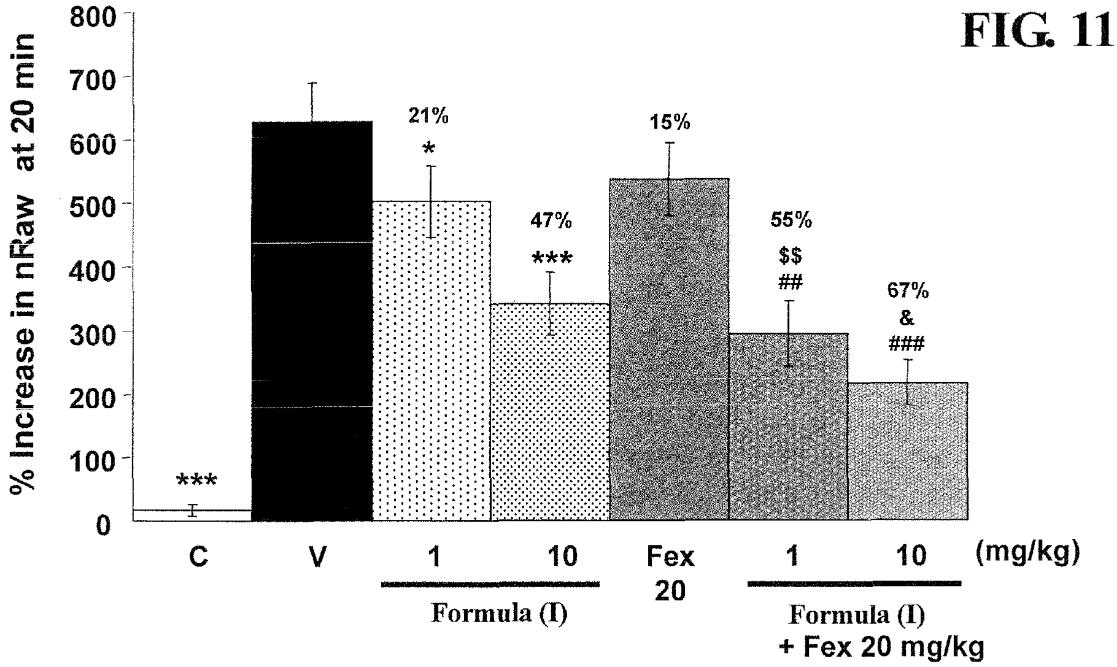


***: p<0.001, *: p<0.05 (vs. vehicle by t-test)
###: p<0.001, ##: p<0.01 (vs. fexofenadine HCl 20 mg/kg by t-test)
$$: p<0.01 (vs. Formula (I) at 1 mg/kg by t-test)
&: p<0.05 (vs. Formula (I) at 10 mg/kg by t-test)

[Figure 12]

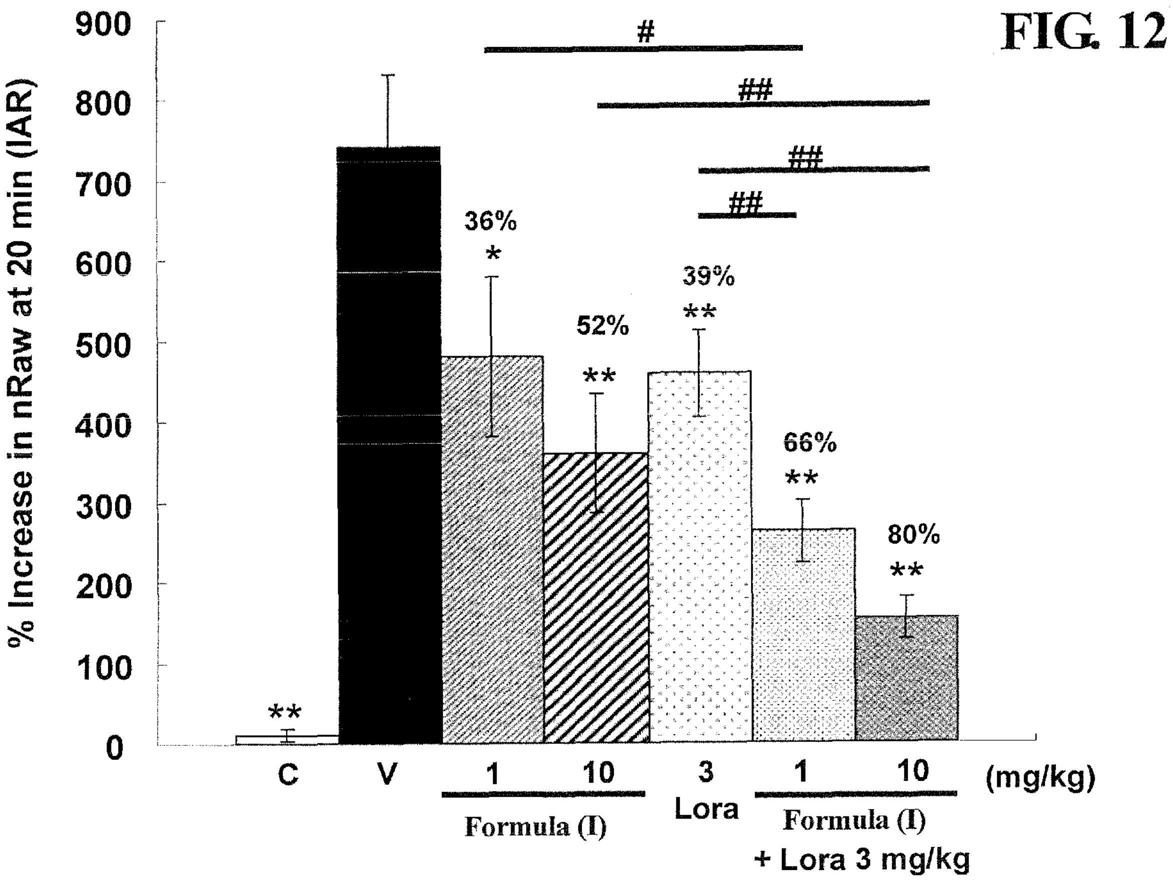


**: p < 0.01, *: p < 0.05 (vs. vehicle by t-test)
##: p < 0.01, #: p < 0.05 (t-test)

[Figure 13]

FIG. 13

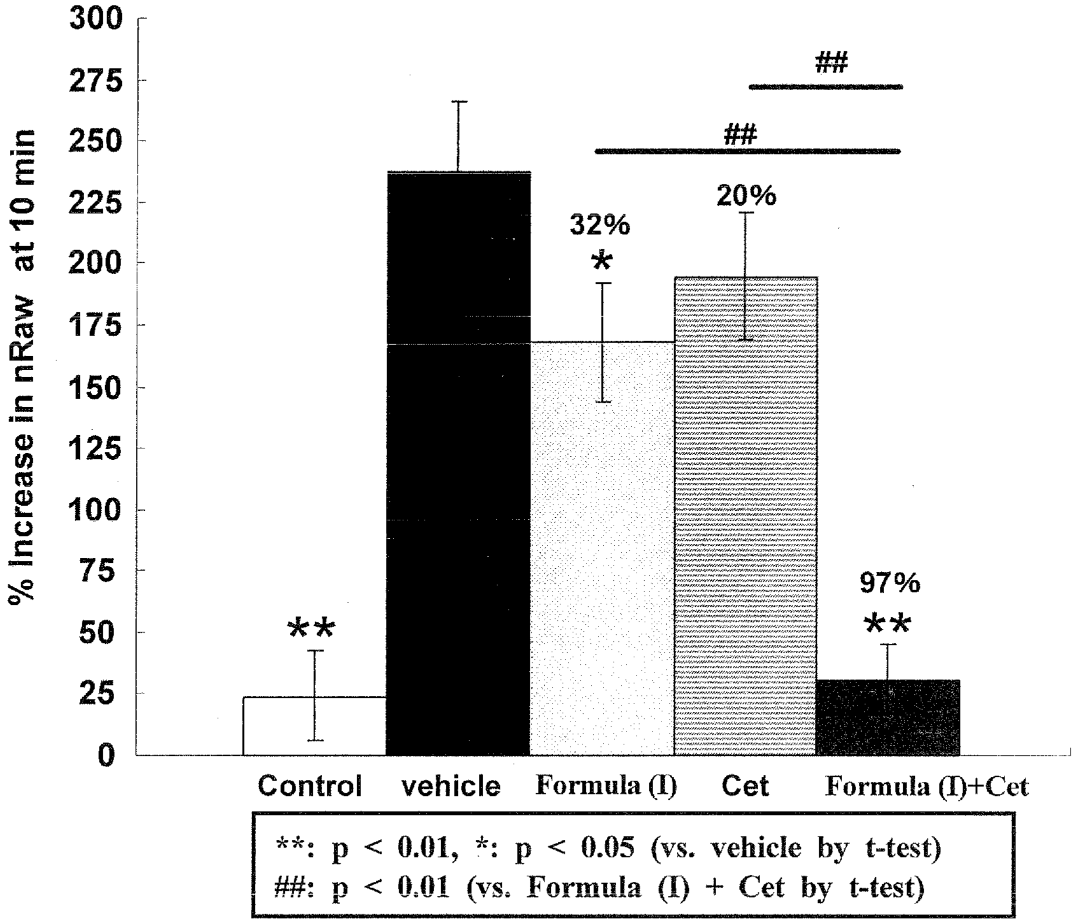

## INTERNATIONAL SEARCH REPORT

International application No.
PCT/JP2012/075030

A. CLASSIFICATION OF SUBJECT MATTER
*A61K31/496*(2006.01)i, *A61K31/445*(2006.01)i, *A61K31/4545*(2006.01)i, *A61K31/495*(2006.01)i, *A61P11/02*(2006.01)i, *A61P37/08*(2006.01)i, *A61P43/00*(2006.01)i

According to International Patent Classification (IPC) or to both national classification and IPC

B. FIELDS SEARCHED

Minimum documentation searched (classification system followed by classification symbols)
A61K31/496, A61K31/445, A61K31/4545, A61K31/495, A61P11/02, A61P37/08, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
CA/REGISTRY(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| Y | JP 2003-530426 A (Merck Frosst Canada and Co.), 14 October 2003 (14.10.2003), claims; paragraphs [0019], [0023]; fig. 1 & US 2003/0055077 A1 & US 2001/0051624 A1 & EP 1274457 A & WO 2001/078697 A2 | 1-8,11-15 |
| Y | WO 2007/037187 A1 (Shionogi & Co., Ltd.), 05 April 2007 (05.04.2007), claims; paragraph [0024]; compound no.II-74; test examples 1 to 2 & US 2010/0063040 A1 & US 2010/0069638 A1 & EP 1939175 A1 & EP 2407453 A1 & EP 2407459 A1 & KR 2008050436 A & CN 101273013 A | 1-8,11-15 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 December, 2012 (04.12.12) | 18 December, 2012 (18.12.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2012/075030

**Box No. II Observations where certain claims were found unsearchable (Continuation of item 2 of first sheet)**

This international search report has not been established in respect of certain claims under Article 17(2)(a) for the following reasons:

1. [x] Claims Nos.: 9-10
because they relate to subject matter not required to be searched by this Authority, namely:
Claims 9-10 pertain to method for treatment of the human body or animal body by surgery or therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying additional fees, this Authority did not invite payment of additional fees.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**

[ ] The additional search fees were accompanied by the applicant's protest and, where applicable, the payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007037187 A
- WO 2006118169 A
- WO 2001078697 A
- WO 2008123349 A
- US 4525358 A
- US 4254129 A
- US 4282233 A


### Non-patent literature cited in the description


- *Journal of Asthma,* 2009, vol. 46, 878-883
- *Journal of Allergy and Clinical Immunology,* 2000, vol. 127 (5), 917-922
- *Journal of Allergy and Clinical Immunology,* 2000, vol. 127 (5), 917-922